# EUROPEAN PATENT APPLICATION

(11) **EP 4 846 928 A1**
(43) Date of publication of application: **02.12.2026**
(21) Application number: 25831012.7
(22) Date of filing: 18.04.2025
(51) Int. Cl.: A61B 5/00, A61B 5/1455, A61B 5/024

(54) **ELECTRONIC DEVICE FOR OBTAINING BIOMETRIC INFORMATION ON BASIS OF PRESENCE OF CONTACT OF USER BODY, AND METHOD FOR CONTROLLING SAME**

(30) Priority: 19.06.2024 KR 20240079670; 09.07.2024 KR 20240090351; 25.07.2024 KR 20240098772
(71) Applicant: Samsung Electronics Co., Ltd., Suwon-si, Gyeonggi-do 16677 (KR)
(72) Inventor: EOM, Kunsun, Suwon-si Gyeonggi-do 16677 (KR); MOON, Hyunseok, Suwon-si Gyeonggi-do 16677 (KR); PARK, Jinyoung, Suwon-si Gyeonggi-do 16677 (KR); JUNG, Myounghoon, Suwon-si Gyeonggi-do 16677 (KR)
(74) Representative: Gulde & Partner
(86) International application number: PCT/KR2025/005324
(87) International publication number: WO 2025/263787

(57) **Abstract**

A processor of an electronic device according to the disclosure is configured to, based on first sensing data obtained by at least one of a first photo diode (PD) or a second PD receiving a light emitted from at least one second LED, identify whether a user's body covers a sensor, and based on identifying that the body covers the sensor, control each of a plurality of first LEDs to emit lights of wavelengths different from one another, and based on second sensing data obtained by at least one of the first PD or the second PD receiving the lights of different wavelengths emitted from the plurality of first LEDs, measure biometric information of the user.

## Description

### Technical Field

One or more example embodiments of the disclosure relate to an electronic device that obtains biometric information based on whether there is contact of a user's body, and a control method of the electronic device.

### Background Art

Recently, as electronic technologies have developed, various types of electronic devices are being developed.

In particular, wearable devices that may contact a part of a user's body are being developed and distributed.

A wearable device may contact a part of a user's body and obtain and provide biometric information of the user. Meanwhile, for a wearable device to obtain biometric information more correctly, the wearable device needs to contact a part of a user's body appropriately.

The aforementioned information may be provided as related art aimed at promoting understanding of the disclosure. Any argument or determination may not be raised regarding which of the aforementioned contents may be applied as prior art related to the disclosure.

### DISCLOSURE

### Technical Solution

According to an aspect of an example embodiment, provided is an electronic device including: a display arranged on a front surface of the electronic device; a sensor arranged on a rear surface of the electronic device; a memory storing instructions, and including at least one storage medium; and at least one processor including at least one processing circuitry, wherein the sensor includes: a plurality of first light emitting diodes (LEDs) that are arranged inside a first area including a center of the sensor, and emit lights of wavelengths different from one another; at least one second LED that is arranged between an outside of the first area and an inside of a second area including the center; and a first photo diode (PD) and a second PD that are arranged between the outside of the first area and the inside of the second area, configured to receive lights emitted from the plurality of first LEDs and the at least one second LED and are arranged to be symmetrical to each other based on the center, and wherein the at least one processor is configured to: based on first sensing data obtained by at least one of the first PD or the second PD that receives a light emitted from the at least one second LED, identify whether a body of a user covers the sensor; based on identifying that the body covers the sensor, control each of the plurality of first LEDs to emit the lights of different wavelengths; and based on second sensing data obtained by at least one of the first PD or the second PD that receives the lights of different wavelengths emitted from the plurality of first LEDs, measure biometric information of the user.

The first sensing data may include: a first absorbance obtained based on the first PD that receives the light emitted from the at least one second LED and a second absorbance obtained based on the second PD that receives the light emitted from the at least one second LED, and the at least one processor may be configured to: identify whether the body covers the first PD and the second PD based on at least one of the first absorbance or the second absorbance.

The at least one second LED may include a 2-1 LED arranged in a first direction and a 2-2 LED arranged in a second direction symmetrical to the first direction based on the center, each of the 2-1 LED and the 2-2 LED may emit a light of a red color band or an infrared (IR) band, the first PD may be arranged to be adjacent to the 2-1 LED, and the second PD may be arranged to be adjacent to the 2-2 LED.

The at least one processor may be configured to: based on the first absorbance obtained based on the first PD that receives the light emitted from the 2-1 LED being greater than or equal to a threshold value, identify that the body does not cover the first PD, and provide a feedback thereon; and based on the second absorbance obtained based on the second PD that receives the light emitted from the 2-2 LED being greater than or equal to the threshold value, identify that the body does not cover the second PD, and provide a feedback thereon.

The at least one processor may be configured to: based on identifying that the body does not cover the first PD, provide at least one of a user interface (UI) screen, a sound notification, or a vibration notification indicating at least one of a location of the body or a location of the first PD on the rear surface of the electronic device as the feedback, and/or based on identifying that the body does not cover the second PD, provide at least one of a UI screen, a sound notification, or a vibration notification indicating at least one of a location of the body or a location of the second PD on the rear surface as the feedback.

The at least one processor may be configured to: based on receiving a user instruction to measure the biometric information, control the at least one second LED to emit a light after an elapse of a predetermined time; and while obtaining the first sensing data after the elapse of the predetermined time, inactivate touch detection through the display.

The electronic device may include: a motion sensor, wherein the at least one processor is configured to: based on detecting, through the motion sensor, a rotation of the electronic device such that the rear surface of the electronic device is upward, control the at least one second LED to emit a light after the elapse of the predetermined time; and based on identifying that the body covers the first PD and the second PD, control the plurality of first LEDs to emit the lights.

The at least one processor may be configured to: while obtaining the second sensing data, provide at least one of a UI screen, a sound notification, or a vibration notification indicating a time left until measurement of the biometric information.

The at least one processor may be configured to: measure the biometric information of the user based on the second sensing data; and the biometric information includes anti-oxidation concentration of the body.

The first area may include a range of 2mm from the center, the second area may include a range of 20mm from the center, the at least one second LED, the first PD, and the second PD may be arranged within a range of between 2mm and 20mm from the center, the first PD may be arranged within a range of 6mm from a first LED included in the at least one second LED, and the second PD may be arranged within a range of 6mm from a second LED included in the at least one second LED.

According to an aspect of an example embodiment, provided is a control method of an electronic device, the electronic device including: a display arranged on a front surface of the electronic device; a sensor arranged on a rear surface of the electronic device, and the sensor including: a plurality of first light emitting diodes (LEDs) that are arranged inside a first area including a center of the sensor, and emit lights of wavelengths different from one another, at least one second LED that is arranged between an outside of the first area and an inside of a second area including the center, and a first photo diode (PD) and a second PD that are arranged between the outside of the first area and the inside of the second area, configured to receive lights emitted from the plurality of first LEDs and the at least one second LED, and are arranged to be symmetrical to each other based on the center, the method including: based on first sensing data obtained by at least one of the first PD or the second PD that receives a light emitted from the at least one second LED, identifying whether a body of a user covers the sensor; based on identifying that the body covers the sensor, controlling each of the plurality of first LEDs to emit the lights of different wavelengths; and based on second sensing data obtained by at least one of the first PD or the second PD that receives the lights of different wavelengths emitted from the plurality of first LEDs, measuring biometric information of the user based on the second sensing data.

The first sensing data may include: first absorbance obtained as the first PD received the light emitted from the at least one second LED and second absorbance obtained as the second PD received the light emitted from the at least one second LED, and the identifying whether the body covers the sensor may include: identifying whether the body covers the first PD and the second PD based on at least one of the first absorbance or the second absorbance.

The at least one second LED may include a first LED arranged in a first direction and a second LED arranged in a second direction symmetrical to the first direction based on the center, each of the first LED and the second LED may emit a light of a red or IR band, the first PD may be arranged to be adjacent to the first LED, and the second PD may be arranged to be adjacent to the second LED.

The identifying whether the body covers the sensor may include: based on the first absorbance obtained based on the first PD that receives the light emitted from the first LED being greater than or equal to a threshold value, identifying that the body does not cover the first PD, and providing a feedback thereon; and based on the second absorbance obtained based on the second PD that receives the light emitted from the second LED being greater than or equal to the threshold value, identifying that the body does not cover the second PD, and providing a feedback thereon.

The providing the feedback with respect to the first PD may include: based on identifying that the body does not cover the first PD, providing at least one of a UI screen, a sound notification, or a vibration notification indicating at least one of a location of the body or a location of the first PD on the rear surface of the electronic device as the feedback, and the providing the feedback with respect to the second PD may include: based on identifying that the body does not cover the second PD, providing at least one of a UI screen, a sound notification, or a vibration notification indicating at least one of a location of the body or a location of the second PD on the rear surface as the feedback.

### DESCRIPTION OF DRAWINGS

The above and other aspects, features, and advantages of the disclosure will be more clearly understood from the following detailed description, taken in conjunction with the accompanying drawings.
FIG. 1 illustrates an electronic device according to an embodiment of the disclosure;
FIG. 2 is a block diagram of an electronic device according to an embodiment of the disclosure;
FIG. 3A is a perspective view illustrating a front surface of an electronic device according to an embodiment of the disclosure;
FIG. 3B is a perspective view illustrating a rear surface of an electronic device according to an embodiment of the disclosure;
FIG. 3C is an exploded perspective view of an electronic device according to an embodiment of the disclosure;
FIG. 4 is a block diagram of an electronic device according to an embodiment of the disclosure;
FIG. 5 is a diagram for illustrating a sensor according to an embodiment of the disclosure;
FIG. 6 is a diagram for illustrating a user interface (UI) provided by an electronic device according to a user instruction controlling to measure biometric information according to an embodiment of the disclosure;
FIG. 7 is a diagram for illustrating contact between a user's body and a sensor according to an embodiment of the disclosure;
FIG. 8 is a diagram for illustrating an example in which a user's body does not appropriately cover a sensor according to an embodiment of the disclosure;
FIG. 9 is a diagram for illustrating an example in which a user's body does not appropriately cover a sensor according to an embodiment of the disclosure;
FIG. 10 is a graph for illustrating first sensing data obtained through a plurality of photo diodes (PDs) in a case where a user's body does not cover a sensor according to an embodiment of the disclosure;
FIG. 11 is a graph for illustrating strength information of a reflective light according to contact between a user's body and a sensor according to an embodiment of the disclosure;
FIG. 12 is a graph for illustrating absorbance included in first sensing data according to an embodiment of the disclosure;
FIG. 13 is a flow chart for illustrating a control method of an electronic device according to an embodiment of the disclosure;
FIG. 14 is a flow chart for illustrating a control method of an electronic device according to an embodiment of the disclosure; and
FIG. 15 is a flow chart for illustrating a control method of an electronic device according to an embodiment of the disclosure.

### MODE FOR INVENTION

Hereinafter, the disclosure will be described in detail with reference to the accompanying drawings. With respect to the detailed description of the drawings, identical or similar components may be designated by identical or similar reference numerals.

As terms used in the embodiments of the disclosure, general terms that are currently used widely were selected as far as possible, in consideration of the functions described in the disclosure. However, the terms may vary depending on the intention of those skilled in the art who work in the pertinent field or previous court decisions, or emergence of new technologies, etc. Further, in particular cases, there may be terms that were designated by the applicant on his own, and in such cases, the meaning of the terms will be described in detail in the relevant descriptions in the disclosure. Accordingly, the terms used in the disclosure should be defined based on the meaning of the terms and the overall content of the disclosure, but not just based on the names of the terms.

Also, in this specification, expressions such as "have," "may have," "include," and "may include" denote the existence of such characteristics (e.g., elements such as numbers, functions, operations, and components), and do not exclude the existence of additional characteristics.

In addition, the expression "at least one of A and/or B" should be interpreted to mean any one of "A" or "B" or "A and B."

Further, the expressions "first," "second," and the like used in this specification may be used to describe various elements regardless of any order and/or degree of importance. Also, such expressions are used only to distinguish one element from another element, and are not intended to limit the elements.

Meanwhile, the description in the disclosure that one element (e.g., a first element) is "(operatively or communicatively) coupled with/to" or "connected to" another element (e.g., a second element) should be interpreted to include both the case where the one element is directly coupled to the another element, and the case where the one element is coupled to the another element through still another element (e.g., a third element).

Also, singular expressions include plural expressions, unless defined differently in the context. Further, in the disclosure, terms such as "include" or "consist of" should be construed as designating that there are such characteristics, numbers, steps, operations, elements, components, or a combination thereof described in the specification, but not as excluding in advance the existence or possibility of adding one or more of other characteristics, numbers, steps, operations, elements, components, or a combination thereof.

In addition, in the disclosure, the term "user" may refer to a person who uses an electronic device or a device using an electronic device (e.g., an artificial intelligence electronic device).

Hereinafter, an embodiment of the disclosure will be described in more detail with reference to the accompanying drawings.

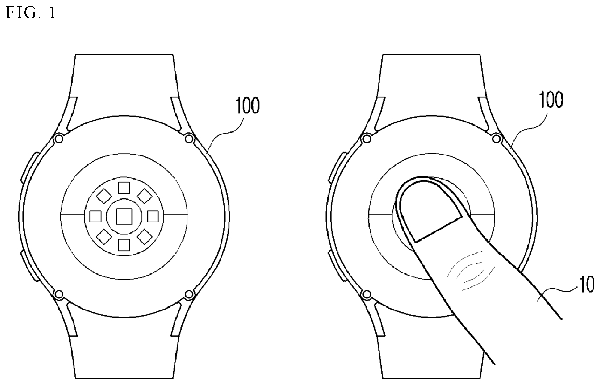

FIG. 1 illustrates an electronic device according to an embodiment of the disclosure.

The electronic device 100 according to various embodiments of the disclosure may include a wearable device and/or a portable device. Here, a wearable device means a device that includes a flexible material (e.g., silicon rubber, fiber), and may be worn by a user, or may contact a part of a user's body. For example, various types of devices such as a watch, clothes, shoes, gloves, glasses, a hat, accessories (e.g., a ring) that may be worn on a body of a person or an animal may be included in wearable devices. However, these are merely examples, and the disclosure is not limited thereto.

According to an embodiment, the electronic device 100 may contact a part of a user's body and measure biometric information of the user. For example, the electronic device 100 may include a photoplethysmogram (PPG) sensor, and measure a heart rate and blood oxygen saturation (SpO2) by using the PPG sensor. Also, the electronic device 100 may measure a physical activity (e.g., a number of steps, a calorie consumption amount, a moving distance), a body temperature, and/or bioelectrical impedance of the user.

According to an embodiment, the electronic device 100 may identify a sleep pattern such as deep sleep, light sleep, and rapid eye movement (REM) sleep based on a heart rate, a physical activity, and/or a body temperature. According to an embodiment, the electronic device 100 may measure impedance by applying a fine electric signal to a human body, and measure a moisture amount and a body fat amount inside the user's body through bioelectrical impedance analysis (BIA). Also, the electronic device 100 may include an electrocardiogram (ECG) measurement sensor, and measure the user's electrocardiogram by using the ECG measurement sensor.

The electronic device 100 according to an embodiment of the disclosure may measure a concentration of an antioxidative component(s) inside the user's body.

Active oxygen is important as a biological protection factor performing a sterilization action of white blood cells, etc., but it is known that excessive generation of active oxygen in vivo may cause various tissue diseases.

Active oxygen is naturally generated in a process of generating energy in vivo (e.g., a metabolic process), and is an important factor in a sterilization action of white blood cells, but excessive generation of active oxygen may damage cells in vivo and deoxyribo nucleic acid (DNA), and may cause chronic diseases and aging.

Antioxidative components neutralize active oxygen, and thereby prevent damage to cells in vivo and aging, and thus it is necessary to appropriately manage the concentration of antioxidative components in vivo by ingesting a sufficient amount of food including antioxidative components such as vitamin E, vitamin C, β-carotene, polyphenol, and microelements (e.g., selenium, copper, zinc).

The electronic device 100 according to an embodiment of the disclosure may induce contact of a finger 10, a palm, a sole, and a forehead wherein antioxidative components are mainly accumulated due to a thick keratin layer on an outside of a body (e.g., the skin) with a sensor, for measuring the concentration of antioxidative components on the outside of the body by a non-invasive method, instead of measuring the concentration of antioxidative components in the blood by accessing the inside of the body by an invasive method.

For example, when the finger 10 contacts the sensor of the electronic device 100, the electronic device 100 may obtain an optical signal by using light emitting diodes (LEDs) and photo diodes (PDs) included in the sensor, and measure physical (or biometric) information of the user, in particular, the concentration of the antioxidative components based on the obtained optical signal.

According to an embodiment, the electronic device 100 may measure the concentration of the antioxidative components through the finger 10 which has a relatively smaller area than a wrist, and thus the finger 10 may not totally cover the sensor, and the concentration of the antioxidative components measured while the finger 10 cannot totally cover the sensor has a problem that its credibility rather deteriorates.

According to an embodiment, the electronic device 100 may identify whether the finger 10 totally covers the sensor and provide a feedback, and measure the concentration of the antioxidative components while the finger 10 totally covers the sensor. Hereinafter, the expression that the sensor is covered by the finger (or the user's body) means that the finger (or the user's body) sufficiently or totally covers the sensor.

For example, if the finger 10 covers only one area of the sensor, and does not cover remaining areas of the sensor, the electronic device 100 may provide a feedback such that, after the user receives the feedback, the finger 10 covers all areas of the sensor (e.g., totally covers the sensor), and if it is identified that the finger 10 covers all areas of the sensor, the electronic device 100 may measure the concentration of the antioxidative components by using the LEDs and the photo diodes (PDs) included in the sensor.

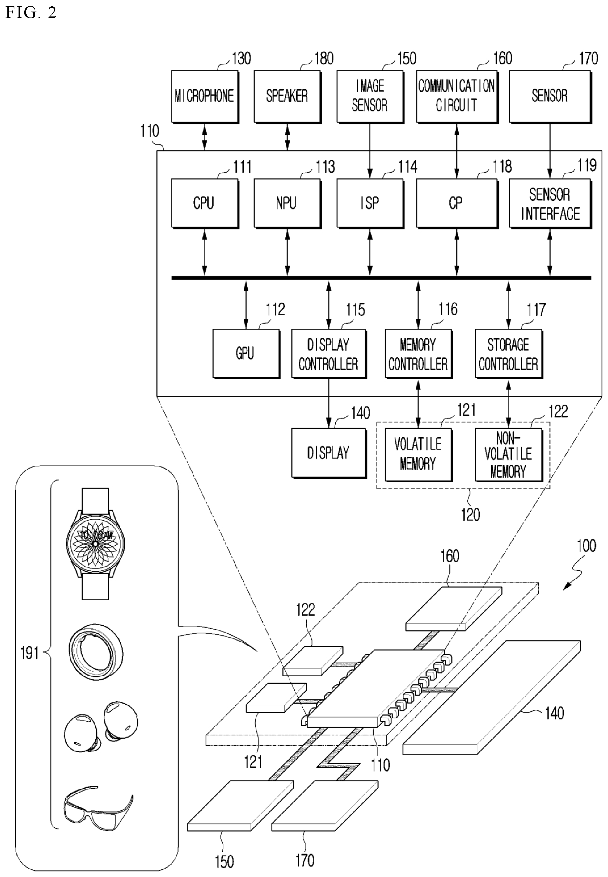

FIG. 2 is a block diagram of an electronic device according to an embodiment of the disclosure.

FIG. 2 is a block diagram of an example of an electronic device that may perform operations according to an embodiment of the disclosure.

Referring to FIG. 2, the electronic device 100 may be implemented in various forms 191 that may be worn by the user such as, for example but not limited to, a smart watch, a smart band, a smart ring, wireless earphones, or smart glasses. Components illustrated in FIG. 2, their relations, and their functions are merely examples, and do not limit implementations explained or claimed in the disclosure. The electronic device 100 may also be referred to as, for example but not limited to, a mobile device, a user device, a multifunctional device, a portable device, or a server.

The electronic device 100 may include components including at least one processor 110, at least one memory 120 (referred to as the memory 120 hereinafter), at least one display 140 (referred to as the display 140 hereinafter), at least one image sensor 150 (referred to as the image sensor 150 hereinafter), at least one communication circuit 160 (referred to as the communication circuit 160 hereinafter), and/or at least one sensor 170 (referred to as the sensor 170 hereinafter). The above components are merely examples. For example, the electronic device 100 may include other components (e.g., power management integrated circuitry (PMIC), audio processing circuitry, an antenna, a rechargeable battery, or an input/output interface). For example, some components may be omitted from the electronic device 100. For example, some components may be integrated into one component.

The at least one processor 110 may be implemented as one or more integrated circuit (or circuitry) (IC) chips, and may execute various types of data processing. The at least one processor 110 may include at least one electric circuit, and perform distributed processing of instructions, programs, and/or data stored in the memory 120 individually and/or collectively. The at least one processor 110 may include a processor assembly including one or more processing circuits. The at least one processor 110 may include any operative processing circuit for controlling the performance and the operations of one or more components of the electronic device 100 (e.g., the memory 120, the microphone 130, the display 140, the image sensor 150, the communication circuit 160, the sensor 170, and/or the speaker 180). For example, the at least one processor 110 (e.g., an application processor (AP)) may be implemented as a system on chip (SoC) (e.g., one chip or chipset). For example, the at least one processor 110 may be implemented as a plurality of cores (or at least one core circuit), a plurality of chips, or a plurality of chipsets. For example, the at least one processor 110 may include one or more processing circuits. For example, the at least one processor 110 may include one or more processing circuits configured to perform several functions of the disclosure individually and/or collectively. As a non-limiting example, at least a portion of the at least one processor 110 may be included in a first chip of the electronic device 100, and at least another portion of the at least one processor 110 may be included in a second chip of the electronic device 100 different from the first chip of the electronic device 100.

For example, the at least one processor 110 may include a central processing unit (CPU) 111, a graphics processing unit (GPU) 112, a neural processing unit (NPU) 113, an image signal processor (ISP) 114, a display controller 115, a memory controller 116, a storage controller 117, a communication processor (CP) 118, and/or a sensor interface 119. These components of the at least one processor 110 are merely examples. For example, the at least one processor 110 may further include other components. For example, some components of the at least one processor 110 may be omitted from the at least one processor 110. For example, some components of the at least one processor 110 may be included as separate components of the electronic device 100 outside the at least one processor 110. For example, some components of the at least one processor 110 (e.g., the memory controller 116) may be included in other components of the electronic device 100 (e.g., at least a portion of the memory 120, an interface (which may be used to connect to at least one component of the electronic device 100), the display 140, and/or the image sensor 150).

The at least one processor 110 may control the other components of the electronic device 100 to perform various operations by executing the instructions stored in the memory 120. The CPU 111(or a central processing circuit) may be configured to control the components of the at least one processor 110 based on execution of instructions stored in the memory 120 (e.g., a volatile memory 121 and/or a non-volatile memory 122). The GPU 112 (or a graphic processing circuit) may be configured to execute parallel operations (e.g., rendering). The NPU 113 (or a neural processing circuit, or an artificial intelligence (AI) chip) may be configured to execute operations for an artificial intelligence model (e.g., convolution computations). The IPS 114 (or an image signal processing circuit) may be configured to process a raw image obtained through the image sensor 150 in a format appropriate for the components inside the electronic device 100 or the components of the at least one processor 110. The display controller 115 (or a display control circuit, or a display processing unit (DPU)) may be configured to process an image obtained from the CPU 111, the GPU 112, the ISP 114, or the memory 120 (e.g., the volatile memory 121) in a format appropriate for the display 140. The memory controller 116 (or a memory control circuit) may be configured to control reading of data from the volatile memory 121 and recording of the data in the volatile memory 121. The storage controller 117 (or a storage control circuit) may be configured to control reading of data from the non-volatile memory 122 and recording of the data in the non-volatile memory 122. The CP 118 (or a communication processing circuit) may be configured to process data obtained from the components of the at least one processor 110 in a format appropriate for being transmitted to another electronic device through the communication circuit 160, and/or process data obtained from another electronic device through the communication circuit 160 in a format appropriate for the components of the at least one processor 110. For example, the communication circuit 160 may include one or more communication circuits. The sensor interface 119 (or a sensing data processing circuit, a sensor hub) may be configured to process data regarding a state of the electronic device 100 and/or a state of a surrounding(s) of the electronic device 100 obtained through the sensor 170 in a format appropriate for the components of the at least one processor 110.

The memory 120 may include one or more storage media (or one or more storage devices). For example, the memory 120 may include a memory assembly including one or more storage media. For example, the one or more storage media may include a permanent memory (e.g., the non-volatile memory 122) such as a hard drive, flash memory, and read-only memory (ROM), a semi-permanent memory (e.g., the volatile memory 121) such as a random access memory (RAM), a storage (or a storage assembly) of any other suitable type, or any combination thereof. The memory 120 may include a cache memory which is a memory of one or more different types that is used for temporarily storing data for the functions or the features of the electronic device 100. As a non-limiting example, the cache memory may be included inside the at least one processor 110. The memory 120 may be fixedly embedded in the electronic device 100, or incorporated into one or more suitable types of components (e.g., a subscriber identity module (SIM) card and/or a secure digital (SD) card) that may be repeatedly inserted into the electronic device 100 and may be removed from the electronic device 100.

For example, the memory 120 may store one or more software applications such as an operating system (or a system) software application, a firmware software application, a driver software application, a plug-in (e.g., an add-in, an add-on, and/or an applet) software application, and/or any other suitable software application. For example, the one or more software applications may include instructions that may be executed by the at least one processor 110. For example, the memory 120 may store instructions that may be called by an application programming interface (API). For example, the memory 120 may store instructions within a library.

The microphone 130 may obtain a sound output from an external object and/or a sound generated from the electronic device 100. According to an embodiment, there may be one or more microphones 130. The speaker 180 may output a sound to the outside. According to an embodiment, there may be one or more speakers 180.

The display 140 may be controlled by the at least one processor 110 (also collectively referred to as "the processor 110"), and output visualized information to the user. The visualized information may include one or more visual objects displayed on the display 140. For example, the one or more visual objects may include a screen, an image, an icon, a graphic user interface (GUI), and user interface (UI) elements. For example, the display 140 may be implemented as, for example but not limited to, a flat panel display (FPD), a curved display, or a flexible display. For example, the display 140 may be implemented as displays in various forms such as, for example but not limited to, a liquid crystal display (LCD), an active matrix organic light emitting diodes (AMOLED) display, light emitting diodes (LEDs), micro LEDs, and mini LEDs.

The display 140 may include a touch detection circuit configured to detect a touch thereon. The touch detection circuit may obtain a user input for the display 140. For example, the touch detection circuit may detect an input (e.g., a touch input or a hovering input) on a specific location by measuring change of a signal (e.g., a voltage, a light amount, a resistance, and/or an electric charge amount) for the specific location of the display 140, and provide information on the detected input to the processor 110.

The communication circuit 160 may perform data communication with other electronic devices by control by the processor 110. For example, the communication circuit 160 may transmit and/or receive control instructions and/or data with other electronic devices. For example, the communication circuit 160 may support transmission and/or reception of electrical signals based on protocols of various types such as, for example but not limited to, Ethernet, a local area network (LAN), a wide area network (WAN), wireless fidelity (Wi-Fi), Bluetooth, Bluetooth Low Energy (BLE), Zigbee, Near Field communication (NFC), ANT+, Cellular (LTE, 5G, 6G, NB-IoT), RFID, ultra wide band (UWB), a global navigation satellite system (GNSS), or radio frequency (RF) communication.

The sensor 170 may generate electric information that may be processed by the processor 110 and/or the memory 120 from non-electric information related to the electronic device 100. The information may also be referred to as sensor data. The sensor 170 may detect an operative state (e.g., the power or the temperature) of the electronic device 100, or the external environmental state (e.g., the user state), and generate electric information corresponding to the detected state.

According to an embodiment, the sensor 170 may include a bio sensor and an electrode sensor.

The bio sensor may be configured to detect biometric information of the user. The biometric information may include various types of data that indicates a physical or physiological state of the user. For example, the bio sensor may include a photoplethysmography (PPG) sensor or a bio marker sensor.

According to an embodiment, the bio sensor may include an emitter and a receiver (or a detector). The bio sensor may output a light to the outside through the emitter under control by the processor 110. Output of a light may be replaced by, for example, expressions such as emission, radiation, and irradiation of a light. The emitter may include a plurality of light emitting elements. For example but not limited thereto, the light emitting elements may be implemented as light emitting diodes (LEDs), laser diodes, or a vertical cavity surface emitting laser (VCSEL). A light output by the emitter may include at least one of an infrared ray (IR), a visible ray, or an ultraviolet (UV) ray. The emitter may include light emitting elements for outputting lights corresponding to infrared rays, visible rays, and/or UV rays.

A light output from the emitter may be irradiated on the user's skin. The user's skin may include various body parts contacted by the bio sensor. For example, the body parts may include a palm or a sole wherein a thickness of an outer layer of the skin is thick, an area wherein venous blood or capillary blood is located, and a finger, a toe, or an earlobe which is a part wherein the density of blood vessels in vivo is high. Also, the body parts may include a wrist, a finger, and the inside of an ear that may be contacted by the bio sensor when the electronic device 100 is worn.

At least some of the irradiated light may be scattered or reflected by the user's body (e.g., the skin, the skin tissue, the layer of fat, the vein, the artery, or the capillary). The receiver may receive the scattered or reflected light, and convert the received light into an electric signal. For example, the receiver may include at least one photo diode (PD) and a phototransistor. However, the disclosure is not limited thereto, and the receiver may be implemented as a complementary metal-oxide semiconductor (CMOS) image sensor or a charge-coupled device (CCD) image sensor. As a non-limiting example, the bio sensor may include an amplifier for amplifying an electric signal and an analog to digital converter (ADC) for converting an electric signal into a digital signal.

According to an embodiment, the bio sensor may measure the biometric information of the user based on a received light under control by the processor 110.

For example, the processor 110 may output a visible ray (e.g., a green light, a red light, or a blue light) or an infrared ray on the skin of the user, and when the output light is reflected by a blood vessel and received, measure an amount of the light that is reflected or absorbed based on the received light and obtain a PPG signal, and obtain a heart rate (HR), oxygen saturation (SpO2), a blood pressure, a blood amount, and a stress index by using the PPG signal.

For example, the processor 110 may output a light of a specific wavelength on the skin of the user, and when the output light is reflected by the skin or a blood vessel of the user and received, analyze the received light and obtain information on a specific substance (or a specific component) inside the skin or the blood vessel of the body. For example, the processor 110 may measure a concentration of the specific substance by using the data obtained by the bio sensor. The substance may include, for example, an antioxidative substance including carotenoid, glucose, urea, lactate, triglyceride, total protein, cholesterol, or ethanol, but the disclosure is not limited thereto. Hereinafter, explanation will be described by suggesting an antioxidative substance as an example, for the convenience of explanation.

The electrode sensor may be configured to detect biometric information through contact with the user's body. For example, the electrode sensor may include at least one electrode. The electrode sensor may detect an electric signal from the body through an electrode contacted with the body under control by the processor 110, and measure an electrocardiogram (ECG), an electromyogram (EMG), and an electroencephalogram (EEG).

According to an embodiment, the sensor 170 may include an illumination sensor, an acceleration sensor, a gyro sensor, a geomagnetic sensor, a barometer, or a temperature sensor.

The illumination sensor may detect a brightness of an external light. For example, the processor 110 may control a brightness of the display 140 by using sensor data detected by the illumination sensor. The acceleration sensor may detect acceleration or an impact due to a movement of the electronic device 100 and/or a movement of the user who uses or owns the electronic device 100. The gyro sensor may detect a rotation direction and/or a rotation angle of the electronic device 100 due to a movement of the electronic device 100 or a movement of the user who uses or owns the electronic device 100. The geomagnetic sensor may detect a direction of geomagnetism. For example, the processor 110 may identify an action (or a movement) of the user by using sensor data detected by the acceleration sensor, the gyro sensor, or the geomagnetic sensor. The barometer may detect atmospheric pressure. For example, the processor 110 may obtain altitude information of the electronic device 100 by using sensor data detected by the barometer. The temperature sensor may measure a temperature of the body by a contact or a non-contact method. For example, the processor 110 may obtain temperature information of the user by using sensor data detected by the temperature sensor.

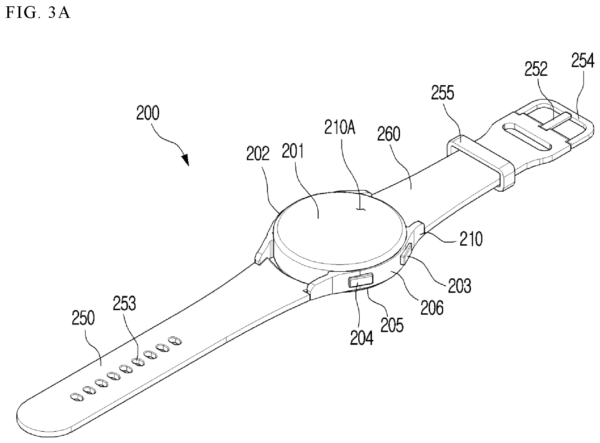

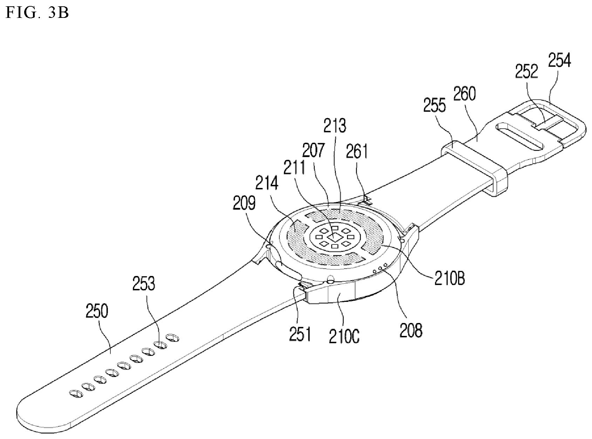

FIG. 3A is a perspective view illustrating a front surface of an electronic device according to an embodiment of the disclosure, and FIG. 3B is a perspective view illustrating a rear surface of an electronic device according to an embodiment of the disclosure.

Referring to FIG. 3A and FIG. 3B, an electronic device 200 according to an embodiment (e.g., the electronic device 101 in FIG. 1) may include a housing 210 including a first surface (or a front surface) 210A, a second surface (or a rear surface) 210B, and a side surface 210C surrounding a space between the first surface 210A and the second surface 210B, and binding elements 250, 260 that are connected to at least a portion of the housing 210 and are configured to detachably bind the electronic device 200 to a portion of the user's body (e.g., a wrist or an ankle). According to another embodiment (not shown), the housing may also refer to a structure forming some of the first surface 210A, the second surface 210B, and the side surface 210C in FIG. 3A and FIG. 3B. According to an embodiment, the first surface 210A may be formed by or include a front surface plate 201 (e.g., a glass plate including various coating layers, and/or a polymer plate) wherein at least a portion is substantially transparent. The second surface 210B may be formed by or include a rear surface plate 207 that is substantially opaque. The rear surface plate 207 may be formed, for example, by coated or colored glass, ceramic, polymer, metal (e.g., aluminum, stainless steel (STS), or magnesium), or a combination of at least two of these substances. The side surface 210C may be formed by or include a side surface bezel structure (or "a side surface element") 206 that is coupled with the front surface plate 201 and the rear surface plate 207, and includes metal and/or a polymer. In some embodiments, the rear surface plate 207 and the side surface bezel structure 206 may be formed as an integrated body, and may include the same substance (e.g., a metallic substance such as aluminum). The binding elements 250, 260 may be formed with various materials and forms, for example but not limited to, fabric, leather, rubber, urethane, metal, ceramic, or a combination of at least two of these substances. Accordingly, the binding elements 250, 260 of an integrated type or a plurality of unit links to be movable with respect to one another may be formed.

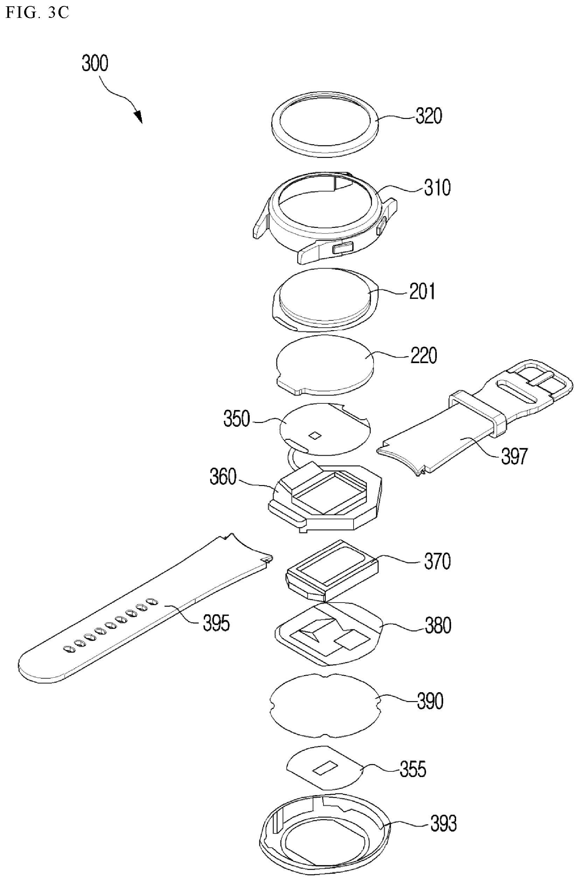

According to an embodiment, the electronic device 200 may include at least one of a display 220 (refer to FIG. 3), audio modules 205, 208, a sensor module 211, key input devices 202, 203, 204, or a connector hole 209. In some embodiments, in the electronic device 200, at least one among the above-described components (e.g., the key input devices 202, 203, 204, the connector hole 209, or the sensor module 211) may be omitted, or other components may be additionally included.

The display 220 may be, for example, visually exposed through a substantial portion of the front surface plate 201. The display 220 may be in a shape corresponding to a shape of the front surface plate 201, and may be various shapes such as a circle, an oval, or a polygon. The display 220 may be coupled with the touch detection circuit, a pressure sensor that may measure the strength (or the pressure) of a touch, and/or a fingerprint sensor, or may be arranged to be adjacent to them.

The audio modules 205, 208 may include a microphone hole 205 and a speaker hole 208. In the microphone hole 205, a microphone for obtaining sounds (e.g., the microphone 130 in FIG. 2) may be arranged on an inside of the electronic device 200, and in some embodiments, a plurality of microphones may be arranged so as to be able to detect directions of sounds. The speaker hole 208 may be used as an external speaker and a receiver for calls. In some embodiments, the speaker hole 208 and the microphone hole 205 may be implemented as one hole, or a speaker (e.g., the speaker 180 in FIG. 2) may be implemented without the speaker hole 208 (e.g., a piezo speaker).

The sensor 211 (e.g., the sensor 170 in FIG. 2) may generate an electric signal or a data value corresponding to an operative state inside the electronic device 200, or an external environmental state. The sensor 211 may include, for example, a bio sensor 211(e.g., an HRM sensor) arranged on the second surface 210B of the housing 210. The electronic device 200 may further include a sensor (not shown), for example but not limited to, at least one of a gesture sensor, a gyro sensor, a barometer, a magnetic sensor, an acceleration sensor, a grip sensor, a color sensor, an infrared (IR) sensor, a bio sensor, a temperature sensor, a humidity sensor, or an illumination sensor.

The sensor 211 may include electrode areas 213, 214 that form a portion of the surface (e.g., second surface 210B) of the electronic device 200, and a bio signal detection circuit (not shown) that is electrically connected with the electrode areas 213, 214. For example, the electrode areas 213, 214 may include a first electrode area 213 and a second electrode area 214 arranged on the second surface 210B of the housing 210. The sensor 211 may be configured such that the electrode areas 213, 214 obtain an electric signal from a portion of the user's body, and the bio signal detection circuit detects the biometric information of the user based on the electric signal.

The key input devices 202, 203, 204 may include a wheel key 202 that is arranged on the first surface 210A of the housing 210 and may rotate in at least one direction, and/or side key buttons 203, 204 that are arranged on the side surface 210C of the housing 210. The wheel key may be in a shape corresponding to the shape of the front surface plate 201. According to another embodiment, the electronic device 200 may not include some or all of the key input devices 202, 203, 204 mentioned above, and the key input devices 202, 203, 204 that are not included may be implemented in a different form such as a soft key on the display 220. The connector hole 209 may accommodate a connector (e.g., a USB connector) for transmitting and receiving power and/or data with an external electronic device, and include another connector hole (not shown) that may accommodate a connector for transmitting and receiving audio signals with an external electronic device. The electronic device 200 may further include, for example, a connector cover (not shown) that covers at least a portion of the connector hole 209, and blocks introduction of foreign substances for the connector hole.

The binding elements 250, 260 may be detachably bound to at least some areas of the housing 210 by using locking elements 251, 261. The binding elements 250, 260 may include one or more of a fixing element 252, a fixing element fastening hole 253, a band guide element 254, and a band fixing ring 255.

The fixing element 252 may be configured to fix the housing 210 and the binding elements 250, 260 to a portion of the user's body (e.g., a wrist or an ankle). The fixing element fastening hole 253 may fix the housing 210 and the binding elements 250, 260 to a portion of the user's body correspondingly to the fixing element 252. The band guide element 254 may be configured to limit a range of movement of the fixing element 252 when the fixing element 252 is fastened to the fixing element fastening hole 253, and may thereby make the binding elements 250, 260 adhesively bound to a portion of the user's body. The band fixing ring 255 may limit the range of movement of the binding elements 250, 260 while the fixing element 252 and the fixing element fastening hole 253 are fastened.

FIG. 3C is an exploded perspective view of an electronic device according to an embodiment of the disclosure.

Referring to FIG. 3C, an electronic device 300 (e.g., the electronic device 101 in FIG. 1, or the electronic device 200 in FIG. 3A to FIG. 3B) may include a side surface bezel structure 310, a wheel key 320 (e.g., the wheel key 202 in FIG. 3A and FIG. 3B), a front surface plate 201, a display 220, a first antenna 350, a second antenna 355, a support element 360 (e.g., a bracket), a battery 370, a printed circuit board 380, a sealing element 390, a rear surface plate 393 (e.g., the rear surface plate 207 in FIG. 3A and FIG. 3B), and binding elements 395, 397 (e.g., the binding elements 250, 260 in FIG. 3A and FIG. 3B). At least one of the components of the electronic device 300 may be identical or similar to at least one of the components of the electronic device 100 in FIG. 1, and/or the components of the electronic device 200 in FIG. 3A to FIG. 3B, and thus overlapping explanation will be omitted below. The support element 360 may be arranged inside the electronic device 300 and connected with the side surface bezel structure 310, or may be formed as an integrated type with the side surface bezel structure 310. The support element 360 may be formed of or include, for example, a metallic material and/or a non-metallic (e.g., a polymer) material. In the support element 360, the display 220 may be coupled to one surface, and the printed circuit board 380 may be coupled to the other surface. On the printed circuit board 380, a processor (e.g., the processor 110 in FIG. 2), memory (e.g., the memory 120 in FIG. 2), and/or an interface may be mounted. The processor may include, for example, one or more of a central processing unit (CPU), a graphics processing unit (GPU), an application processor, a sensor processor, and a communicator processor.

The memory may include, for example, a volatile memory (e.g., the volatile memory 121 in FIG. 2) or a non-volatile memory (e.g., the non-volatile memory 122 in FIG. 2). The interface may include, for example but not limited to, a high definition multimedia interface (HDMI), a universal serial bus (USB) interface, an SD card interface, and/or an audio interface. The interface may, for example but not limited to, electrically or physically connect the electronic device 300 to an external electronic device, and include a USB connector, an SD card and/or a multimedia Card (MMC) connector, or an audio connector.

The battery 370 may be a device for supplying power to at least one component of the electronic device 300, and may include, for example, a primary cell that cannot be recharged, or a secondary cell that may be recharged, or a fuel cell. At least a portion of the battery 370 may be arranged, for example, on a substantially identical plane to the printed circuit board 380. The battery 370 may be arranged integrally inside the electronic device 300, or arranged to be detachable from the electronic device 300.

The first antenna 350 may be arranged between the display 220 and the support element 360. The first antenna 350 may include, for example but not limited to, a near field communication (NFC) antenna, a wireless charging antenna, and/or a magnetic secure transmission (MST) antenna. The first antenna 350 may, for example, perform near field communication with an external device, and/or transmit and receive power needed for charging wirelessly, and send a near field communication signal and/or a magnetism-based signal including payment data. According to another embodiment, an antenna structure may be formed by or include a portion of the side surface bezel structure 310 and/or the support element 360, or a combination thereof.

The second antenna 355 may be arranged between the printed circuit board 380 and the rear surface plate 393. The second antenna 355 may include, for example but not limited to, a near field communication (NFC) antenna, a wireless charging antenna, and/or a magnetic secure transmission (MST) antenna. The second antenna 355 may, for example, perform near field communication with an external device, or transmit and receive power needed for charging wirelessly, and send a near field communication signal or a magnetism-based signal including payment data. According to another embodiment, an antenna structure may be formed by or include a portion of the side surface bezel structure 310 and/or the rear surface plate 393, or a combination thereof.

The sealing element 390 may be located between the side surface bezel structure 310 and the rear surface plate 393. The sealing element 390 may be configured to block moisture and a foreign substance that are introduced from the outside into a space surrounded by the side surface bezel structure 310 and the rear surface plate 393.

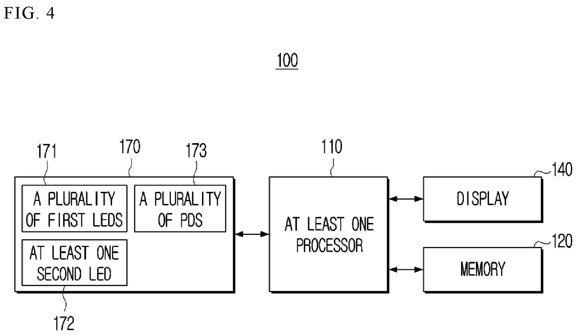

FIG. 4 is a block diagram of an electronic device according to an embodiment of the disclosure.

Referring to FIG. 4, the electronic device 100 may include a memory 120, a display 140, a sensor 170, and at least one processor 110 (referred to as the processor 110 hereinafter). Accordingly, explanation overlapping with FIG. 2 will be omitted.

According to an embodiment, the processor 110 may be implemented as, for example but not limited to, a digital signal processor (DSP) processing digital signals, a microprocessor, and a timing controller (T-CON). However, the disclosure is not limited thereto, and the processor 110 may include, for example but not limited to, one or more of a central processing unit (CPU), a micro controller unit (MCU), a micro processing unit (MPU), a controller, an application processor (AP) or a communication processor (CP), an ARM processor, and an artificial intelligence (AI) processor, or may be defined by the terms. Also, the processor 110 may be implemented as a system on chip (SoC) having a processing algorithm stored therein or large scale integration (LSI), or implemented in the form of a field programmable gate array (FPGA). The processor 110 may perform various functions by executing computer executable instructions stored in the memory 120.

The processor 110 may include, for example but not limited to, one or more of a central processing unit (CPU), a graphics processing unit (GPU), an accelerated processing unit (APU), a many integrated core (MIC), a digital signal processor (DSP), a neural processing unit (NPU), a hardware accelerator, or a machine learning accelerator. The processor 110 may control one or a random combination of the other components of the electronic device 100, and perform an operation related to communication or data processing. Also, the processor 110 may execute one or more programs or instructions stored in the memory 120. For example, the processor 110 may perform the method according to an embodiment of the disclosure by executing the one or more instructions stored in the memory 120.

In a case where the method according to an embodiment of the disclosure includes a plurality of operations, the plurality of operations may be performed by one processor, or performed by a plurality of processors. For example, when a first operation, a second operation, and a third operation are performed by the method according to an embodiment, all of the first operation, the second operation, and the third operation may be performed by a first processor, or the first operation and the second operation may be performed by the first processor (e.g., a generic-purpose processor), and the third operation may be performed by a second processor (e.g., an artificial intelligence-dedicated processor).

The processor 110 may be implemented as a single core processor including one core, or may be implemented as one or more multicore processors including a plurality of cores (e.g., multicores of the same kind or multicores of different kinds). In a case where the processor 110 is implemented as multicore processors, each of the plurality of cores included in the multicore processors may include an internal memory of the processor such as a cache memory, an on-chip memory, etc., and a common cache shared by the plurality of cores may be included in the multicore processors. Also, each of the plurality of cores (or some of the plurality of cores) included in the multicore processors may independently read a program instruction for implementing the method according to an embodiment of the disclosure and perform the instruction, or the plurality of entire cores (or some of the cores) may be linked with one another, and read a program instruction for implementing the method according to an embodiment of the disclosure and perform the instruction.

In a case in which the method according to an embodiment of the disclosure includes a plurality of operations, the plurality of operations may be performed by one core among the plurality of cores included in the multicore processors, or they may be performed by the plurality of cores. For example, when the first operation, the second operation, and the third operation are performed by the method according to an embodiment, all of the first operation, the second operation, and the third operation may be performed by a first core included in the multicore processors, or the first operation and the second operation may be performed by the first core included in the multicore processors, and the third operation may be performed by a second core included in the multicore processors.

In the embodiments of the disclosure, the processor may mean a system on chip (SoC) wherein at least one processor and other electronic components are integrated, a single core processor, a multicore processor, or a core included in the single core processor or the multicore processor. Also, here, the core may be implemented as a CPU, a GPU, an APU, a MIC, a DSP, an NPU, a hardware accelerator, or a machine learning accelerator, etc., but the embodiments of the disclosure are not limited thereto.

According to an embodiment, the sensor 170 may include a plurality of first LEDs 171. For example, the plurality of first LEDs 171 may be arranged inside a first area including a center of the sensor 170.

According to an embodiment, the first area may be a circle within a first distance from the center. For example, the first area may be a circular area to a distance of 2mm from the center. However, 2mm is merely an example of the first distance for the convenience of explanation, and the disclosure is not limited thereto.

According to an embodiment, each of the plurality of first LEDs 171 may emit lights of different wavelengths from one another.

According to an embodiment, the sensor 170 may include at least one second LED 172. For example, the at least one second LED 172 may be arranged between an outside of the first area and an inside of a second area including the center. According to an embodiment, the at least one second LED 172 may emit a light of a red or an IR band.

According to an embodiment, the second area may be a circle within a second distance from the center, and the second distance may be greater than the first distance. For example, the second area may be a circular area defined by points that have a distance of 20mm from the center. However, 20mm is merely an example of the second distance for the convenience of explanation, and the disclosure is not limited thereto.

According to an embodiment, the first area in a circular form and the second area in a circular form may be concentric circles.

According to an embodiment, a plurality of photo diodes (PDs) 173 may be arranged between the outside of the first area and the inside of the second area. For example, each of the plurality of PDs 173 may receive lights emitted by the plurality of first LEDs 171 and lights emitted by the at least one second LED 172.

For example, each of the plurality of PDs 173 may receive (or, detect) lights that were emitted by the plurality of first LEDs 171 and were reflected on or passed through the user's body (e.g., the finger 10).

According to an embodiment, the plurality of first LEDs 171 and the plurality of PDs 173 may constitute a plethysmography (PPG) sensor. For example, each of the plurality of first LEDs 171 may emit lights of different wavelengths (e.g., a light of a green wavelength, a light of a red wavelength, a light of an infrared wavelength) from one another, and the plurality of PDs 173 may receive the lights that were emitted by the plurality of first LEDs 171 and were reflected on or passed through the user's body (e.g., a wrist, the finger 10) that contacts the sensor 170. According to an embodiment, the processor 110 may identify a strength of lights reflected on the user's body (or, absorbance (a strength of lights that were absorbed into the user's body)) based on a strength of the lights received by the plurality of PDs 173.

According to an embodiment, when lights of wavelengths different from one another emitted by the plurality of first LEDs 171 are reflected on or pass through the user's body and are detected (or, received) by the plurality of PDs 173, the processor 110 may measure an absorbance spectrum from the lights detected by the plurality of PDs 173, and identify the concentration of carotenoid which is an antioxidative component based on the absorbance spectrum.

According to an embodiment, for identifying the concentration of antioxidative components with higher credibility, the processor 110 may identify whether the user's body (e.g., the finger 10) totally covers the sensor 170 before controlling the plurality of first LEDs 171 to emit lights.

For example, the processor 110 may control the at least one second LED 172 to emit lights. When lights emitted by the at least one second LED 172 are reflected on or pass through the user's body and are detected (or, received) by the plurality of PDs 173, the plurality of PDs 173 may obtain sensing data (referred to as first sensing data hereinafter). The processor 110 may identify whether the user's body covers the sensor 170 based on the first sensing data.

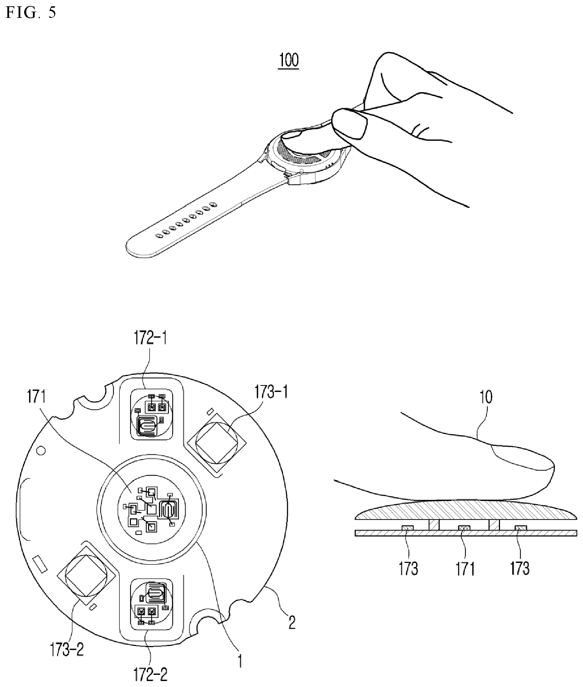

FIG. 5 is a diagram for illustrating a sensor according to an embodiment of the disclosure.

According to an embodiment, the sensor 170 may include a plurality of first LEDs 171 arranged inside the first area 1.

The sensor 170 may include at least one second LED 172 arranged between the outside of the first area 1 and the inside of the second area 2.

For example, the at least one second LED 172 may include a 2-1 LED 172-1 and a 2-2 LED 172-2, and each of the 2-1 LED 172-1 and the 2-2 LED 172-2 may be arranged between the outside of the first area 1 and the inside of the second area 2.

The sensor 170 may include a plurality of PDs 173 arranged between the outside of the first area 1 and the inside of the second area 2. For example, the plurality of PDs 173 may include a first PD 173-1 and a second PD 173-2, and the first PD 173-1 and the second PD 173-2 may be arranged to be substantially symmetrical to each other based on a center of the first area 1 and/or the second area 2.

According to an embodiment, the first PD 173-1 may be arranged to be adjacent to the 2-1 LED 172-1, and the second PD 173-2 may be arranged to be adjacent to the 2-2 LED 172-2. For example, the first PD 173-1 may be arranged within a range of 6mm from the 2-1 LED 172-1, and the second PD 173-2 may be arranged within a range of 6mm from the 2-2 LED 172-2.

However, this is merely an example for the convenience of explanation, and in order to avoid crosstalk in which a light emitted by the 2-2 LED 172-2 is mainly received by the first PD 173-1, or a light emitted by the 2-1 LED 172-1 is mainly received by the second PD 173-2, the first PD 173-1 may be arranged to be adjacent to the 2-1 LED 172-1, and the second PD 173-2 may be arranged to be adjacent to the 2-2 LED 172-2. For example, the first PD 173-1 and the second PD 173-2 may be arranged such that a light emitted by the 2-1 LED 172-1 is mainly received by the first PD 173-1, and a light emitted by the 2-2 LED 172-2 is mainly received by the second PD 173-2.

According to an embodiment, the processor 110 may control the at least one second LED 172 to emit lights. Some of the lights emitted by the at least one second LED 172 may be reflected on the user's body that contacts the sensor 170 and received by at least one of the first PD 173-1 or the second PD 173-2, and the remaining lights emitted by the at least one second LED 172 may be absorbed into the user's body.

According to an embodiment, the processor 110 may identify whether the user's finger 10 covers an area corresponding to the first PD 173-1 in the sensor 170 based on the strength of the light absorbed by the first PD 173-1 (or, the light received by the first PD 173-1) among the lights emitted by the at least one second LED 172.

For example, the processor 110 may identify whether the finger 10 covers an area corresponding to the first PD 173-1 based on the strength of the light absorbed by the first PD 173-1 arranged to be adjacent to the 2-1 LED 172-1 in the light emitted by the 2-1 LED 172-1.

According to an embodiment, the first sensing data may include the strength of the light received by the first PD 173-1 (referred to as the strength of a first reflective light or first absorbance hereinafter).

According to an embodiment, the strength of the first reflective light may include the strength of the light that was emitted by the 2-1 LED 172-1 and was reflected on the user's body and absorbed by the first PD 173-1, and as the user's body covers the area corresponding to the first PD 173-1 more appropriately, the strength of the first reflective light may be increased, and as the user's body covers the area corresponding to the first PD 173-1 less appropriately, the strength of the first reflective light may be decreased.

According to an embodiment, the strength of the first reflective light may be in an inverse-proportional relation to the first absorbance.

According to an embodiment, the first absorbance may be a value indicating a degree that the user's body covering the sensor 170 absorbs a light emitted by the 2-1 LED 172-1, and as the user's body covers the sensor 170 less appropriately, the value of the first absorbance may be increased, and as the user's body covers the sensor 170 more appropriately, the value of the first absorbance may be decreased.

According to an embodiment, if the first absorbance is greater than or equal to a threshold value, the processor 110 may identify that the user's body does not cover the area corresponding to the first PD 173-1 that obtained the first absorbance, and if the first absorbance is smaller than the threshold value, the processor 110 may identify that the user's body covers the area corresponding to the first PD 173-1.

According to an embodiment, if it is identified that the user's body does not cover the area corresponding to the first PD 173-1, the processor 110 may provide a feedback such that the user's body covers the first PD 173-1 after receiving the feedback.

According to an embodiment, the processor 110 may identify whether the finger 10 covers the area corresponding to the second PD 173-2 in the sensor 170 based on the strength of the light absorbed by the second PD 173-2 (or, the light received by the second PD 173-2) among the lights emitted by the at least one second LED 172.

For example, the processor 110 may identify whether the finger 10 covers an area corresponding to the second PD 173-2 based on the strength of the light absorbed by the second PD 173-2 arranged to be adjacent to the 2-2 LED 172-2 in the light emitted by the 2-2 LED 172-2.

According to an embodiment, the first sensing data may include the strength of the light received by the second PD 173-2 (referred to as the strength of a second reflective light or second absorbance hereinafter).

According to an embodiment, the strength of the second reflective light may be in an inverse-proportional relation to the second absorbance.

According to an embodiment, if the second absorbance is greater than or equal to the threshold value, the processor 110 may identify that the user's body does not cover the area corresponding to the second PD 173-2 that obtained the second absorbance, and if the second absorbance is smaller than the threshold value, the processor 110 may identify that the user's body covers the area corresponding to the second PD 173-2.

According to an embodiment, if it is identified that the user's body does not cover the area corresponding to the second PD 173-2, the processor 110 may provide a feedback such that the user's body covers the second PD 173-2.

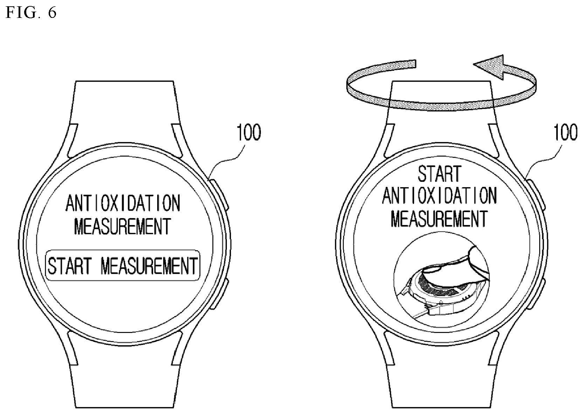

According to an embodiment of the disclosure, if a user instruction controlling the electronic device 100 to measure biometric information is received, the processor 110 may guide the user's body (e.g., the user's finger 10) to cover the sensor 170, and control the at least one second LED 172 to emit lights. Detailed explanation in this regard will be described with reference to FIG. 6.

FIG. 6 is a diagram for illustrating a user interface (UI) provided by an electronic device according to a user instruction controlling to measure biometric information according to an embodiment of the disclosure.

Referring to FIG. 6, if a user instruction controlling the electronic device 100 to measure biometric information of the user, e.g., the concentration of antioxidative components is received, the processor 110 may display a UI that guides (or, induces) contact between the sensor 170 and the user's body.

For example, antioxidative components are mainly accumulated in the finger 10 and a palm rather than in a wrist, and thus the concentration may be measured with relatively higher credibility in the case of contact between the sensor 170 and the finger 10 than in the case of contact between the sensor 170 and a wrist.

As the finger 10 is a relatively smaller area than a wrist, in order for the finger 10 to cover the sensor, the processor 110 according to an embodiment may display a UI element (e.g., text and/or image) that guides an appropriate position of the finger 10. For example, the processor 110 may sequentially display a UI that guides a rotation of the electronic device 100 such that the sensor 170 is upward, and a UI that guides a position of the finger 10 such that the finger 10 totally covers the sensor 170.

For example, if a user instruction controlling the electronic device 100 to measure biometric information is received, the processor 110 may inactivate the touch detection circuit of the display 140 during a predetermined time, or ignore the user's touch.

For example, after a user instruction controlling the electronic device 100 to measure biometric information (e.g., the concentration of antioxidative components) is received, a touch not intended by the user may be input according to the user's action for rotating the electronic device 100 during a predetermined time.

According to an embodiment, when a user instruction is received, the processor 110 may not perform an operation corresponding to a touch not intended by the user by inactivating the touch detection circuit during the predetermined time, or ignoring the user's touch.

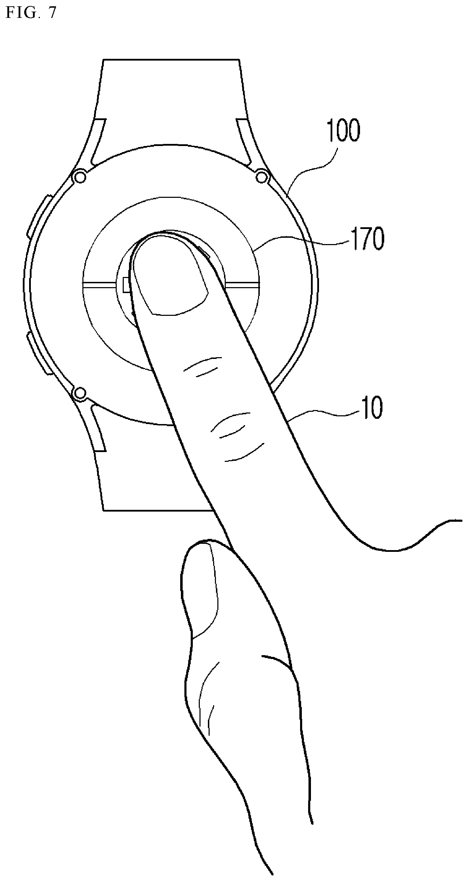

According to an embodiment, when a user instruction is received, the processor 110 may control the at least one second LED 172 to emit lights after a predetermined time passes. Detailed explanation in this regard will be described with reference to FIG. 7.

FIG. 7 is a diagram for illustrating contact between a user's body and a sensor according to an embodiment of the disclosure.

Referring to FIG. 7, if a user instruction controlling to measure biometric information is received, the processor 110 may inactivate touch detection including, for example, ignoring the user's touch during the predetermined time, or inactivating the touch detection circuit.

According to an embodiment, the processor 110 may identify whether the user's body (e.g., the finger 10) covers the sensor 170 by controlling the at least one second LED 172 to emit lights after the predetermined time passes.

For example, the processor 110 may identify whether the finger 10 covers each of the area corresponding to the first PD 173-1 and the area corresponding to the second PD 173-2 based on the first sensing data including the first absorbance obtained as the first PD 173-1 receives some of the light emitted by the 2-1 LED 172-1 and the second absorbance obtained as the second PD 173-2 receives some of the light emitted by the 2-2 LED 172-2.

According to an embodiment, if it is identified that the finger 10 covers each of the area corresponding to the first PD 173-1 and the area corresponding to the second PD 173-2, the processor 110 may control the plurality of first LEDs 171 to emit lights of different wavelengths from one another for obtaining the biometric information.

According to an embodiment, if second sensing data is obtained as the first PD 173-1 and the second PD 173-2 receive the lights of different wavelengths from one another emitted by the plurality of first LEDs 171, the processor 110 may measure the biometric information of the user based on the second sensing data.

For example, for measuring the antioxidative components accumulated on the skin unevenly with reproducibility, the plurality of first LEDs 171 may be arranged inside the first area 1 including the center of the sensor 170, and the first PD 173-1 and the second PD 173-2 may be arranged to be substantially symmetrical to each other based on the center.

The plurality of first LEDs 171 and the first PD 173-1 may be arranged to be distanced to be appropriate for securing an optical path (or path length), and the plurality of first LEDs 171 and the second PD 173-2 may be also arranged to be distanced to be appropriate for securing an optical path.

According to an embodiment, the electronic device 100 may include a motion sensor. For example, the motion sensor may detect a movement of the electronic device 100.

For example, the motion sensor may include an infrared (IR) sensor, an ultrasonic sensor, an optical sensor, and a gyroscope sensor.

According to an embodiment, if rotation of the electronic device 100 for the rear surface of the electronic device 100 (e.g., 210B in FIG. 3B) to be upward is detected through the motion sensor, the processor 110 may control the at least one second LED 172 to emit lights after the predetermined time passes.

For example, the predetermined time may include a time required for the user's body (e.g., the finger 10) to move to cover the sensor 170 (e.g., 5 seconds).

According to an embodiment, after the predetermined time passes, the processor 110 may control the at least one second LED 172 to emit lights for identifying whether the user's body covers the sensor 170, and after the at least one second LED 172 emits lights, the processor 110 may inactivate touch detection of the display 140 while the plurality of PDs 173 obtain the first sensing data by receiving the lights.

According to an embodiment, the processor 110 may obtain the first sensing data after the predetermined time passes, and if it is identified that the user's body covers the sensor 170 based on the first sensing data, the processor 110 may display a UI screen indicating a time that is left until measurement of the biometric information, and/or provide a sound notification and/or a vibration notification.

According to an embodiment, if it is identified that the user's body covers the sensor 170 based on the first sensing data, the processor 110 may control the plurality of first LEDs 171 to emit lights of different wavelengths from one another, and after the plurality of first LEDs 171 emit lights, the plurality of PDs 173 may obtain the second sensing data by receiving the lights.

The processor 110 may measure the biometric information based on the second sensing data.

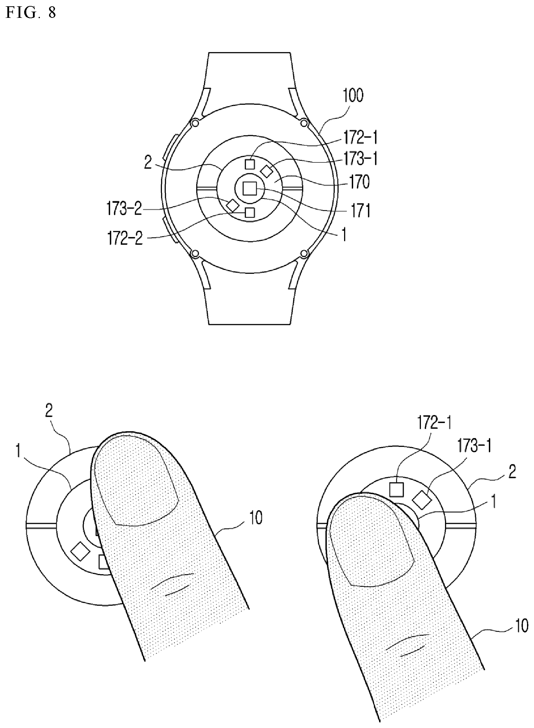

FIG. 8 is a diagram for illustrating an example in which a user's body does not appropriately cover a sensor according to an embodiment of the disclosure.

Referring to FIG. 8, the sensor 170 may include a 2-1 LED 172-1 and a 2-2 LED 172-2 arranged between the outside of the first area 1 and the inside of the second area 2, and include a first PD 173-1 arranged to be adjacent to the 2-1 LED 172-1 and a second PD 173-2 arranged to be adjacent to the 2-2 LED 172-2.

According to an embodiment, the first PD 173-1 and the second PD 173-2 may be electrically separated from each other.

According to an embodiment, a case in which contact between the user's finger 10 and the sensor 170 is poor (or, a case in which the finger 10 does not cover the sensor 170) may include a case in which the finger 10 covers the 2-1 LED 172-1 and the first PD 173-1, and does not totally cover the 2-2 LED 172-2 and the second PD 173-2.

However, this is merely an example, and the disclosure is not limited thereto. For example, the above case may include a case in which the finger 10 covers the 2-2 LED 172-2 and the second PD 173-2, and does not totally cover the 2-1 LED 172-1 and the first PD 173-1.

According to an embodiment, the processor 110 may identify whether the finger 10 covers the sensor 170, e.g., all of the 2-1 LED 172-1, the 2-2 LED 172-2, the first PD 173-1, and the second PD 173-2 included in the sensor 170, and if the finger 10 does not cover one of components of the sensor 170, e.g., does not cover the first PD 173-1 or does not cover the second PD 173-2, the processor 110 may provide a feedback indicating the same.

For example, if the first absorbance obtained as the first PD 173-1 receives some of the light emitted by the 2-1 LED 172-1 is greater than or equal to the threshold value, the processor 110 may identify that the finger 10 does not cover the first PD 173-1. The processor 110 may provide a feedback such that the finger 10 covers the first PD 173-1.

For example, the processor 110 may output a sound that guides a movement of the finger 10 (e.g., a sound uttering 'Please move your finger little more to the upper right.'), an image (e.g., an image showing that the finger moves to the upper right), or a vibration indicating a poor contact between the sensor 170 and the user's body as a feedback, based on the position of the first PD 173-1 in the sensor 170 (e.g., the upper right).

For example, if the second absorbance obtained as the second PD 173-2 receives some of the light emitted by the 2-2 LED 172-2 is greater than or equal to the threshold value, the processor 110 may identify that the finger 10 does not cover the second PD 173-2. The processor 110 may provide a feedback such that the finger 10 covers the second PD 173-2.

For example, the processor 110 may output a sound that guides a movement of the finger 10 (e.g., a sound uttering 'Please move your finger little more to the lower left.'), an image (e.g., an image showing that the finger moves to the lower left), or a vibration indicating a poor contact between the sensor 170 and the user's body as a feedback, based on the position of the second PD 173-2 in the sensor 170 (e.g., the lower left).

In the aforementioned example, it is described that the at least one second LED 172 includes the 2-1 LED 172-1 and the 2-2 LED 172-2, and the plurality of PDs 173 include the first PD 173-1 adjacent to the 2-1 LED 172-1 and the second PD 173-2 adjacent to the 2-2 LED 172-2, but the disclosure is not limited thereto.

For example, in the sensor 170, the at least one second LED 172 arranged between the outside of the first area and the inside of the second area 2 may further include a 2-3 LED and a 2-4 LED, and the plurality of PDs 173 may further include a third PD and a fourth PD.

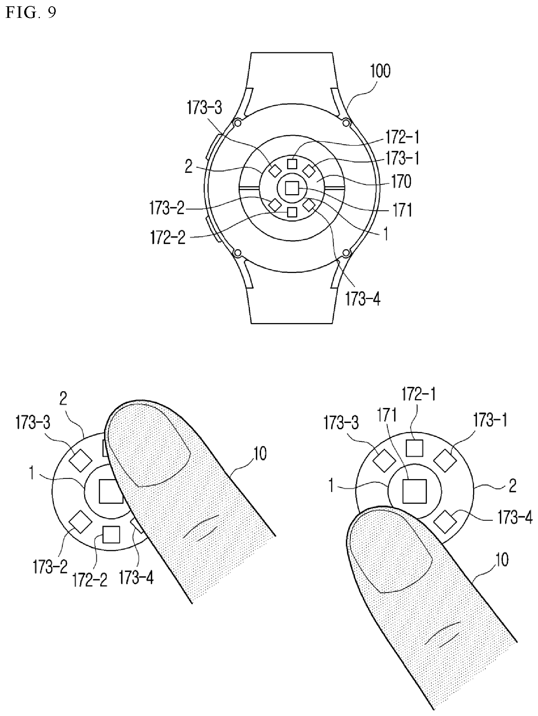

FIG. 9 is a diagram for illustrating an example in which a user's body does not appropriately cover a sensor according to an embodiment of the disclosure.

Referring to FIG. 9, the sensor 170 may include a 2-1 LED 172-1 and a 2-2 LED 172-2 arranged between the outside of the first area 1 and the inside of the second area 2, and include a first PD 173-1 and a third PD 173-3 arranged to be adjacent to the 2-1 LED 172-1 and a second PD 173-2 and a fourth PD 173-4 arranged to be adjacent to the 2-2 LED 172-2.

Before the plurality of first LEDs 171 emit lights, the at least one second LED 172 may emit lights according to control by the processor 110, and the processor 110 may identify whether the user's finger 10 covers the sensor 170 based on a first absorbance obtained by the first PD 173-1, a second absorbance obtained by the second PD 173-2, a third absorbance obtained by the third PD 173-3, and a fourth absorbance obtained by the fourth PD 173-4.

For example, a case in which contact between the user's finger 10 and the sensor 170 is poor (or, a case in which the finger 10 does not cover the sensor 170) may include a case in which the finger 10 does not cover at least one of the first PD 173-1 to the fourth PD 173-4.

For example, referring to FIG. 9, if the first absorbance obtained by the first PD 173-1 is smaller than the threshold value, and each of the second absorbance obtained by the second PD 173-2, the third absorbance obtained by the third PD 173-3, and the fourth absorbance obtained by the fourth PD 173-4 is greater than or equal to the threshold value, the processor 110 may identify that the finger 10 covers the area corresponding to the first PD 173-1, but does not cover the areas corresponding to each of the second PD 173-2, the third PD 173-3, and the fourth PD 173-4.

According to an embodiment, the processor 110 may output a sound that guides a movement of the finger 10 (e.g., a sound uttering 'Please move your finger little more to the left.'), an image (e.g., an image showing that the finger moves to the left), or a vibration indicating poor contact between the sensor 170 and the user's body as a feedback such that the finger 10 covers the areas corresponding to each of the second PD 173-2, the third PD 173-3, and the fourth PD 173-4.

For example, referring to FIG. 9, if the second absorbance obtained by the second PD 173-2 is smaller than the threshold value, and each of the first absorbance obtained by the first PD 173-1, the third absorbance obtained by the third PD 173-3, and the fourth absorbance obtained by the fourth PD 173-4 is greater than or equal to the threshold value, the processor 110 may identify that the finger 10 covers the area corresponding to the second PD 173-2, but does not cover the areas corresponding to each of the first PD 173-1, the third PD 173-3, and the fourth PD 173-4.

According to an embodiment, the processor 110 may output a sound that guides a movement of the finger 10 (e.g., a sound uttering 'Please move your finger little more to the upper right.'), an image (e.g., an image showing that the finger moves to the upper right), or a vibration indicating poor contact between the sensor 170 and the user's body as a feedback such that the finger 10 covers the areas corresponding to each of the first PD 173-1, the third PD 173-3, and the fourth PD 173-4.

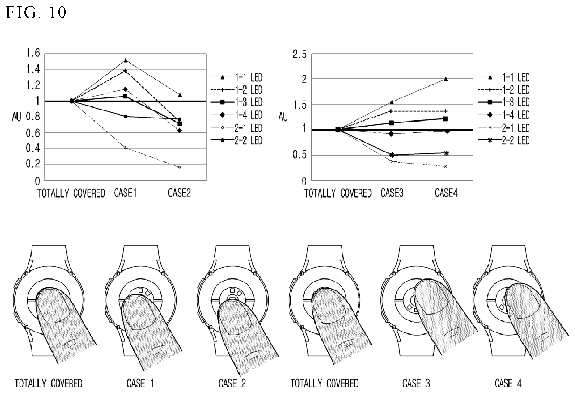

FIG. 10 is a graph for illustrating first sensing data obtained through the plurality of PDs 173 in a case where a user's body does not cover a sensor according to an embodiment of the disclosure.

Referring to FIG. 10, if the finger 10 totally covers the plurality of PDs 173 included in the sensor 170, the processor 110 may assume the strength of reflective lights obtained by each of the plurality of PDs 173 as a reference value. Here, in FIG. 10, the reference value is assumed as '1' for the convenience of explanation, but the disclosure is not limited thereto.

CASE 1 and CASE 2 in FIG. 10 assume a case in which the user's finger 10 does not cover the first PD 173-1, but covers the second PD 173-2 among the plurality of PDs 173.

According to an embodiment, the plurality of first LEDs 171 may include a 1-1 LED, a 1-2 LED, a 1-3 LED, and a 1-4 LED. For example, each of the 1-1 LED, the 1-2 LED, the 1-3 LED, and the 1-4 LED may emit lights of different wavelengths from one another. However, this is merely an example for the convenience of explanation, and the disclosure is not limited thereto.

For example, comparing the CASE 1 and the CASE 2, the strength of the first reflective light obtained as the first PD 173-1 receives some of the light emitted by the 1-4 LED included in the plurality of first LEDs 171 is greater than or equal to the reference value in the CASE 1, and is smaller than the reference value in the CASE 2, and thus there is no consistency. Alternatively, the strength of the first reflective light obtained as the first PD 173-1 receives some of the light emitted by the 1-1 LED included in the plurality of first LEDs 171 is greater than or equal to the reference value.

According to an embodiment, the processor 110 cannot identify whether the finger 10 covers the first PD 173-1 based on the strength of lights emitted by the plurality of first LEDs 171 arranged in a different area from the plurality of PDs 173 (e.g., the inside of the first area 1) and were received by the first PD 173-1 (e.g., the strength of the first reflective light or the first absorbance), because of crosstalk.

Comparing the CASE 1 and the CASE 2, the strength of the first reflective light obtained as the first PD 173-1 receives some of the light emitted by the 2-1 LED 172-1 adjacent to the first PD 173-1 among the at least one second LED 172 arranged between the outside of the first area 1 and the inside of the second area 2 is smaller than the reference value in each of the CASE 1 and the CASE 2, and thus there is consistency.

According to an embodiment, the processor 110 may identify whether the finger 10 covers the first PD 173-1 based on the strength of a light emitted by the 2-1 LED 172-1 adjacent to the first PD 173-1 among the at least one second LED 172 arranged in the same area as the plurality of PDs 173 (e.g., between the outside of the first area 1 and the inside of the second area 2) and was received by the first PD 173-1 (e.g., the strength of the first reflective light or the first absorbance).

For example, if the strength of the first reflective light obtained as the first PD 173-1 receives some of the light emitted by the 2-1 LED 172-1 is smaller than the reference value, the processor 110 may identify that the finger 10 does not cover the first PD 173-1.

CASE 3 and CASE 4 in FIG. 10 assume a case in which the user's finger 10 does not cover the second PD 173-2, but covers the first PD 173-1 among the plurality of PDs 173.

For example, the strength of the second reflective light obtained as the second PD 173-2 receives some of the light emitted by the 1-1 LED included in the plurality of first LEDs 171 is greater than or equal to the reference value. Alternatively, comparing the CASE 3 and the CASE 4, the strength of the second reflective light obtained as the second PD 173-2 receives some of the light emitted by the 1-4 LED included in the plurality of first LEDs 171 is smaller than the reference value in the CASE 3, and is adjacent to the reference value in the CASE 4, and thus there is no consistency.

According to an embodiment, the processor 110 cannot identify whether the finger 10 covers the second PD 173-2 based on the strength of lights emitted by the plurality of first LEDs 171 arranged in a different area from the plurality of PDs 173 (e.g., the inside of the first area 1) and were received by the second PD 173-2 (e.g., the strength of the second reflective light or the second absorbance), because of crosstalk.

Comparing the CASE 3 and the CASE 4, the strength of the second reflective light obtained as the second PD 173-2 receives some of the light emitted by the 2-2 LED 172-2 adjacent to the second PD 173-2 among the at least one second LED 172 arranged between the outside of the first area 1 and the inside of the second area 2 is smaller than the reference value in each of the CASE 3 and the CASE 4, and thus there is consistency.

According to an embodiment, the processor 110 may identify whether the finger 10 covers the second PD 173-2 based on the strength of a light emitted by the 2-2 LED 172-2 adjacent to the second PD 173-2 among the at least one second LED 172 arranged in the same area as the plurality of PDs 173 (e.g., between the outside of the first area 1 and the inside of the second area 2) and was received by the second PD 173-2 (e.g., the strength of the second reflective light or the second absorbance).

For example, if the strength of the second reflective light obtained as the second PD 173-2 receives some of the light emitted by the 2-2 LED 172-2 is smaller than the reference value, the processor 110 may identify that the finger 10 does not cover the second PD 173-2.

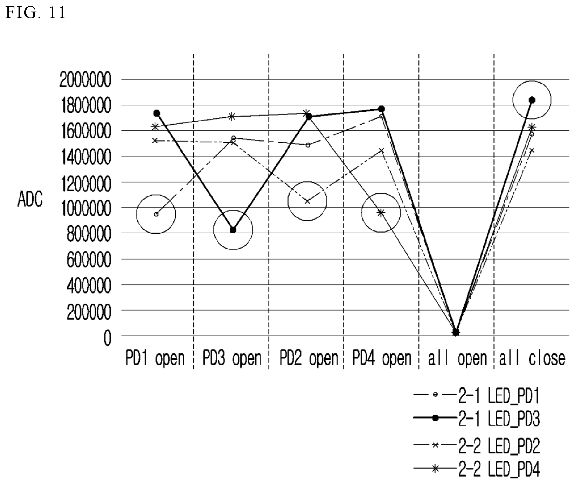

FIG. 11 is a graph for illustrating strength information of a reflective light according to contact between a user's body and a sensor according to an embodiment of the disclosure.

As illustrated in FIG. 9, according to an embodiment, the at least one second LED 172 may include a 2-1 LED 172-1 arranged in a first direction (e.g., a direction of 12 o'clock) and a 2-2 LED 172-2 arranged in a second direction (e.g., a direction of 6 o'clock) symmetrical to the first direction based on the center. According to an embodiment, each of the first PD 173-1 and the third PD 173-3 may be arranged to be adjacent to the 2-1 LED 172-1, and each of the second PD 173-2 and the fourth PD 173-4 may be arranged to be adjacent to the 2-2 LED 172-2.

In the graph in FIG. 11, an X-axis may indicate a state in which the first PD 173-1 is open (exposed or not covered), a state in which the third PD 173-3 is open, a state in which the second PD 173-2 is open, a state in which the fourth PD 173-4 is open, a state in which all of the plurality of PDs 173 are open, and a state in which all of the plurality of PDs 173 are closed (e.g., a state in which the finger 10 covers the plurality of PDs 173), and a Y-axis may indicate the strength of reflective lights that were obtained as each of the plurality of PDs 173 receives lights in each state.

According to an embodiment, in an open state of the first PD 173-1 (e.g., a state in which the finger 10 does not cover only the first PD 173-1), if the 2-1 LED 172-1 adjacent to the first PD 173-1 emits a light, the strength of the first reflective light obtained as the first PD 173-1 receives some of the light emitted by the 2-1 LED 172-1 is smaller than the threshold value, and each of the strength of the second reflective light obtained as the second PD 173-2 receives the light, the strength of the third reflective light obtained as the third PD 173-3 receives the light, and the strength of the fourth reflective light obtained as the fourth PD 173-4 receives the light is greater than or equal to the threshold value, and thus the processor 110 may identify that the finger 10 does not cover the first PD 173-1.

According to an embodiment, in an open state of the third PD 173-3 (e.g., a state in which the finger 10 does not cover only the third PD 173-3), if the 2-1 LED 172-1 adjacent to the third PD 173-3 emits a light, the strength of the third reflective light obtained as the third PD 173-3 receives some of the light emitted by the 2-1 LED 172-1 is smaller than the threshold value, and each of the strength of the first reflective light obtained as the first PD 173-1 receives the light, the strength of the second reflective light obtained as the second PD 173-2 receives the light, and the strength of the fourth reflective light obtained as the fourth PD 173-4 receives the light is greater than or equal to the threshold value, and thus the processor 110 may identify that the finger 10 does not cover the third PD 173-3.

According to an embodiment, in an open state of the second PD 173-2 (e.g., a state in which the finger 10 does not cover only the second PD 173-2), if the 2-2 LED 172-2 adjacent to the second PD 173-2 emits a light, the strength of the second reflective light obtained as the second PD 173-2 receives some of the light emitted by the 2-2 LED 172-2 is smaller than the threshold value, and each of the strength of the first reflective light obtained as the first PD 173-1 receives the light, the strength of the third reflective light obtained as the third PD 173-3 receives the light, and the strength of the fourth reflective light obtained as the fourth PD 173-4 receives the light is greater than or equal to the threshold value, and thus the processor 110 may identify that the finger 10 does not cover the second PD 173-2.

According to an embodiment, in an open state of the fourth PD 173-4 (e.g., a state in which the finger 10 does not cover only the fourth PD 173-4), if the 2-2 LED 172-2 adjacent to the fourth PD 173-4 emits a light, the strength of the fourth reflective light obtained as the fourth PD 173-4 receives some of the light emitted by the 2-2 LED 172-2 is smaller than the threshold value, and each of the strength of the first reflective light obtained as the first PD 173-1 receives the light, the strength of the second reflective light obtained as the second PD 173-2 receives the light, and the strength of the third reflective light obtained as the third PD 173-3 receives the light is greater than or equal to the threshold value, and thus the processor 110 may identify that the finger 10 does not cover the fourth PD 173-4.

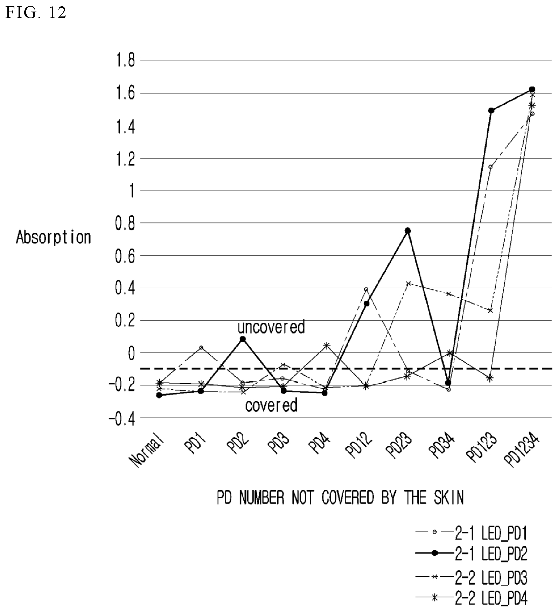

FIG. 12 is a graph for illustrating absorbance included in first sensing data according to an embodiment of the disclosure.

In the graph in FIG. 12, the X-axis may indicate a state in which all of the plurality of PDs 173 are closed (e.g., a state in which the finger 10 covers the plurality of PDs 173) ('normal' in FIG. 12), a state in which the first PD 173-1 is open, a state in which the second PD 173-2 is open, a state in which the third PD 173-3 is open, a state in which the fourth PD 173-4 is open, a state in which the first PD 173-1 and the second PD 173-2 are open, a state in which the second PD 173-2 and the third PD 173-3 are open, a state in which the third PD 173-3 and the fourth PD 173-4 are open, a state in which the first PD 173-1, the second PD 173-2, and the third PD 173-3 are open, and a state in which all of the plurality of PDs 173 are open, and the Y-axis may indicate the absorbance that was obtained as each of the plurality of PDs 173 receives lights in each state.

In FIG. 12, a 2-1 LED 172-1 arranged in the first direction of the sensor 170 and a 2-2 LED 172-2 arranged in a second direction symmetrical to the first direction based on the center of the sensor 170 may be included, for the convenience of explanation.

According to an embodiment, each of the 2-1 LED 172-1 and the 2-2 LED 172-2 may be arranged between the outside of the first area 1 in a circular form including the center and the inside of the second area 2 in a circular form greater than the first area 1. According to an embodiment, the first area 1 and the second area 2 may be concentric circles.

According to an embodiment, the first PD 173-1 and the second PD 173-2 among the plurality of PDs 173 may be arranged to be adjacent to the 2-1 LED 172-1, and the third PD 173-3 and the fourth PD 173-4 may be arranged to be adjacent to the 2-2 LED 172-2.

According to an embodiment, the first PD 173-1 and the fourth PD 173-4 may be arranged to be substantially symmetrical based on the center, and the second PD 173-2 and the fourth PD 173-4 may be arranged to be substantially symmetrical based on the center.

FIG. 12 is a graph for illustrating first sensing data obtained through the plurality of PDs 173 in a case where a user's body does not cover a sensor or covers a sensor according to an embodiment of the disclosure.

Referring to FIG. 12, if the finger 10 totally covers the plurality of PDs 173 included in the sensor 170 (normal), the processor 110 may set a threshold value based on the strength of the lights absorbed into the user's body (referred to as absorbance hereinafter), based on the strength of the lights received by the plurality of PDs 173 which are some of the lights emitted by the at least one second LED 172. Here, in FIG. 12, the threshold value was set as '0' for the convenience of explanation, but the disclosure is not limited thereto. According to an embodiment, the strength of a reflective light and absorbance may be in an inverse-proportional relation.

According to an embodiment, if the finger 10 does not cover the first PD 173-1 among the plurality of PDs 173, but covers the remaining PDs 173-2, 173-3, 173-4, the first absorbance obtained as the first PD 173-1 adjacent to the 2-1 LED 172-1 receives some of the light emitted by the 2-1 LED 172-1 included in the first sensing data exceeds the threshold value (e.g., '0').

According to an embodiment, if the first absorbance obtained as the first PD 173-1 adjacent to the 2-1 LED 172-1 receives some of the light emitted by the 2-1 LED 172-1 is greater than or equal to the threshold value, and the second absorbance obtained as the second PD 173-2 adjacent to the 2-1 LED 172-1 receives some of the light emitted by the 2-1 LED 172-1, the third absorbance obtained as the third PD 173-3 adjacent to the 2-2 LED 172-2 receives some of the light emitted by the 2-2 LED 172-2, and the fourth absorbance obtained by the fourth PD 173-4 adjacent to the 2-2 LED 172-2 by receiving some of the light emitted by the 2-2 LED 172-2 are smaller than the threshold value, the processor 110 may identify that the user's finger 10 does not cover the first PD 173-1, but covers the remaining PDs 173-2, 173-3, 173-4, and provide a feedback such that the user's finger 10 covers the first PD 173-1.

According to an embodiment, if the user's finger 10 does not cover any one PD among the plurality of PDs 173, the processor 110 may identify the any one PD that the finger 10 does not cover based on the absorbance obtained by each of the plurality of PDs 173 in the same manner as the aforementioned example. For example, if the finger 10 does not cover the fourth PD 173-4, but covers the remaining PDs 173-1, 173-2, 173-3, the fourth absorbance obtained as the fourth PD 173-4 adjacent to the 2-2 LED 172-2 receives some of the light emitted by the 2-2 LED 172-2 included in the first sensing data exceeds the threshold value (e.g., '0').

According to an embodiment, if the fourth absorbance obtained as the fourth PD 173-4 adjacent to the 2-2 LED 172-2 receives some of the light emitted by the 2-2 LED 172-2 is greater than or equal to the threshold value, and the first absorbance obtained as the first PD 173-1 adjacent to the 2-1 LED 172-1 receives some of the light emitted by the 2-1 LED 172-1, the second absorbance obtained as the second PD 173-2 adjacent to the 2-1 LED 172-1 receives some of the light emitted by the 2-1 LED 172-1, and the third absorbance obtained as the third PD 173-3 adjacent to the 2-2 LED 172-2 receives some of the light emitted by the 2-2 LED 172-2 are smaller than the threshold value, the processor 110 may identify that the user's finger 10 does not cover the fourth PD 173-4, but covers the remaining PDs 173-1, 173-2, 173-3, and provide a feedback such that the user's finger 10 covers the fourth PD 173-4.

According to an embodiment, if the user's finger 10 does not cover the first PD 173-1, the second PD 173-2, and the third PD 173-3, but covers only the fourth PD 173-4, the first absorbance obtained as the first PD 173-1 adjacent to the 2-1 LED 172-1 receives some of the light emitted by the 2-1 LED 172-1, the second absorbance obtained as the second PD 173-2 adjacent to the 2-1 LED 172-1 receives some of the light emitted by the 2-1 LED 172-1, and the third absorbance obtained as the third PD 173-3 adjacent to the 2-2 LED 172-2 receives some of the light emitted by the 2-2 LED 172-2 included in the first sensing data are greater than or equal to the threshold value, and the fourth absorbance obtained as the fourth PD 173-4 adjacent to the 2-2 LED 172-2 receives some of the light emitted by the 2-2 LED 172-2 is smaller than the threshold value.

According to an embodiment, the processor 110 may identify that the finger 10 does not cover the first PD 173-1, the second PD 173-2, and the third PD 173-3, but covers only the fourth PD 173-4 based on the first absorbance to the fourth absorbance included in the first sensing data, and provide a feedback such that the user's finger 10 covers the first PD 173-1, the second PD 173-2, and the third PD 173-3.

According to the various embodiments of the disclosure, in a case where the finger 10 does not cover any one of the first PD 173-1 to the fourth PD 173-4 as the at least one second LED 172 emits a light of a wavelength of about 660nm, a result of an experiment wherein the processor 110 identified (or, determined) the PD that a part of the user's body (e.g., the finger 10) does not cover among the plurality of PDs 173 based on the first sensing data (e.g., absorbance) is as follows.

**[Table 1]**

| Absorbance (Absorption) | | Determination | | | | | |
|---|---|---|---|---|---|---|---|
| | | Pass | PD1 | PD2 | PD3 | PD4 | Rem |
| Actual | Pass | 39 | 0 | 0 | 0 | 1 | 0 |
| | PD1 | 4 | 76 | 0 | 0 | 0 | 0 |
| | PD2 | 9 | 0 | 71 | 0 | 0 | 0 |
| | PD3 | 6 | 0 | 0 | 73 | 1 | 0 |
| | PD4 | 4 | 0 | 0 | 0 | 76 | 0 |
| | Rem | 4 | 1 | 1 | 1 | 33 | 0 |

In the Table 1, Pass may include a state in which each of the plurality of PDs 173 is appropriately covered by a part of the body, and Rem(remote) may include a state in which a part of the body does not contact the sensor 170, but is located to be adjacent to the sensor 170 (e.g., a state in which the plurality of PDs 173 are not appropriately covered by a part of the body).

Based on the Table 1, the result of the experiment is summed up as follows.

**[Table 2]**

| | | Determination | |
|---|---|---|---|
| | | Positive | Negative |
| Actual | Positive | 97.5% | 2.5% |
| | Negative | 7.5% | 92.5% |

For example, when a part of the user's body appropriately covers the sensor 170, cases where the processor 110 determines that the user's body appropriately covers the sensor 170 (Actual: positive - Determination: positive) may be 97.5%, and when a part of the user's body does not cover a part of the sensor 170, cases where the processor 110 determines that the user's body does not cover a part of the sensor 170 (Actual: negative - Determination: negative) may be 92.5%.

According to the various embodiments of the disclosure, in a case where the finger 10 does not cover any one of the first PD 173-1 to the fourth PD 173-4 as the at least one second LED 172 emits a light of a wavelength of about 660nm, a result of an experiment wherein the processor 110 identified (or, determined) the PD that a part of the user's body (e.g., the finger 10) does not cover among the plurality of PDs 173 based on the strength of a reflective light is as in the following Table 3.

**[Table 3]**

| Strength of Reflective Light (Raw Intensity) | | Determination | | | | | |
|---|---|---|---|---|---|---|---|
| | | Pass | PD1 | PD2 | PD3 | PD4 | Rem |
| | Pass | 36 | 0 | 0 | 2 | 2 | 0 |
| | PD1 | 10 | 53 | 16 | 1 | 0 | 0 |
| Actual | PD2 | 7 | 0 | 35 | 20 | 18 | 0 |
| | PD3 | 8 | 18 | 18 | 33 | 3 | 0 |
| | PD4 | 4 | 0 | 0 | 20 | 56 | 0 |
| | Rem | 4 | 0 | 1 | 1 | 34 | 0 |

Based on Table 4, the result of the experiment is summed up as follows.

**[Table 4]**

| | | Determination | |
|---|---|---|---|
| | | Positive | Negative |
| Actual | Positive | 90.0% | 10.0% |
| | Negative | 9.2% | 90.8% |

For example, when a part of the user's body appropriately covers the sensor 170, cases where the processor 110 determines that the user's body appropriately covers the sensor 170 (Actual: positive - Determination: positive) may be 90.0%, and when a part of the user's body does not cover a part of the sensor 170, cases where the processor 110 determines that the user's body does not cover a part of the sensor 170 (Actual: negative - Determination: negative) may be 90.8%.

According to the various embodiments of the disclosure, in a case where the finger 10 does not cover any one of the first PD 173-1 to the fourth PD 173-4 as the at least one second LED 172 emits a light of a wavelength of about 940nm, a result of an experiment wherein the processor 110 identified (or, determined) the PD that a part of the user's body (e.g., the finger 10) does not cover among the plurality of PDs 173 based on the first sensing data (e.g., absorbance) is as follows.

**[Table 5]**

| Absorbance (Absorption) | | Determination | | | | | |
|---|---|---|---|---|---|---|---|
| | | Pass | PD1 | PD2 | PD3 | PD4 | Rem |
| | Pass | 40 | 0 | 0 | 0 | 0 | 0 |
| | PD1 | 0 | 80 | 0 | 0 | 0 | 0 |
| Actual | PD2 | 2 | 0 | 78 | 0 | 0 | 0 |
| | PD3 | 2 | 0 | 0 | 78 | 0 | 0 |
| | PD4 | 1 | 0 | 0 | 0 | 79 | 0 |
| | Rem | 2 | 33 | 1 | 2 | 3 | 0 |

In the Table 5, Pass may include a state in which each of the plurality of PDs 173 is appropriately covered by a part of the body, and Rem(remote) may include a state in which a part of the body does not contact the sensor 170, but is located to be adjacent to the sensor 170 (e.g., a state in which the plurality of PDs 173 are not appropriately covered by a part of the body).

Based on the Table 5, the result of the experiment is summed up as follows.

**[Table 6]**

| | | Determination | |
|---|---|---|---|
| | | Positive | Negative |
| Actual | Positive | 100% | 0.0% |
| | Negative | 1.9% | 98.1% |

For example, when a part of the user's body appropriately covers the sensor 170, cases where the processor 110 determines that the user's body appropriately covers the sensor 170 (Actual: positive - Determination: positive) may be 100%, and when a part of the user's body does not cover a part of the sensor 170, cases where the processor 110 determines that the user's body does not cover a part of the sensor 170 (Actual: negative - Determination: negative) may be 98.1%.

According to the various embodiments of the disclosure, in a case where the finger 10 does not cover any one of the first PD 173-1 to the fourth PD 173-4 as the at least one second LED 172 emits a light of a wavelength of about 940nm, a result of an experiment wherein the processor 110 identified (or, determined) the PD that a part of the user's body (e.g., the finger 10) does not cover among the plurality of PDs 173 based on the strength of a reflective light is as in the following Table 7.

**[Table 7]**

| Strength of Reflective Light (Raw Intensity) | | Determination | | | | | |
|---|---|---|---|---|---|---|---|
| | | Pass | PD1 | PD2 | PD3 | PD4 | Rem |
| | Pass | 38 | 0 | 0 | 0 | 2 | 0 |
| | PD1 | 1 | 60 | 19 | 0 | 0 | 0 |
| Actual | PD2 | 0 | 0 | 40 | 20 | 20 | 0 |
| | PD3 | 2 | 20 | 20 | 38 | 0 | 0 |
| | PD4 | 1 | 0 | 0 | 20 | 59 | 0 |
| | Rem | 2 | 37 | 0 | 0 | 1 | 0 |

Based on the Table 7, the result of the experiment is summed up as follows.

**[Table 8]**

| | Determination | | |
|---|---|---|---|
| | Positive | Negative | |
| Actual | Positive | 95.0% | 5.0% |
| | Negative | 1.7% | 98.3% |

For example, when a part of the user's body appropriately covers the sensor 170, cases where the processor 110 determines that the user's body appropriately covers the sensor 170 (Actual: positive - Determination: positive) may be 95.0%, and when a part of the user's body does not cover a part of the sensor 170, cases where the processor 110 determines that the user's body does not cover a part of the sensor 170 (Actual: negative - Determination: negative) may be 98.3%.

According to the various embodiments of the disclosure, in a case where the finger 10 does not cover any one of the first PD 173-1 to the fourth PD 173-4 as the at least one second LED 172 emits a light by combining a light of a wavelength of about 660nm and a light of a wavelength of about 940nm, a result of an experiment wherein the processor 110 identified (or, determined) the PD that a part of the user's body (e.g., the finger 10) does not cover among the plurality of PDs 173 based on the first sensing data is as follows.

**[Table 9]**

| Absorbance (Absorption) | | Determination | | | | | |
|---|---|---|---|---|---|---|---|
| | | Pass | PD1 | PD2 | PD3 | PD4 | Rem |
| | Pass | 35 | 0 | 0 | 2 | 3 | 0 |
| | PD1 | 30 | 33 | 16 | 1 | 0 | 0 |
| Actual | PD2 | 21 | 0 | 30 | 17 | 12 | 0 |
| | PD3 | 18 | 18 | 18 | 24 | 2 | 0 |
| | PD4 | 23 | 0 | 0 | 18 | 39 | 0 |
| | Rem | 8 | 0 | 3 | 1 | 10 | 18 |

Based on the Table 9, the result of the experiment is summed up as follows.

**[Table 10]**

| | | Determination | |
|---|---|---|---|
| | | Positive | Negative |
| Actual | Positive | 87.5% | 12.5% |
| | Negative | 27.8% | 72.2% |

For example, when a part of the user's body appropriately covers the sensor 170, cases where the processor 110 determines that the user's body appropriately covers the sensor 170 (Actual: positive - Determination: positive) may be 87.5%, and when a part of the user's body does not cover a part of the sensor 170, cases where the processor 110 determines that the user's body does not cover a part of the sensor 170 (Actual: negative - Determination: negative) may be 72.2%.

According to the various embodiments of the disclosure, in a case where the finger 10 does not cover any one of the first PD 173-1 to the fourth PD 173-4 as the at least one second LED 172 emits a light by combining a light of a wavelength of about 660nm and a light of a wavelength of about 940nm, a result of an experiment wherein the processor 110 identified (or, determined) the PD that a part of the user's body (e.g., the finger 10) does not cover among the plurality of PDs 173 based on the strength of a reflective light is as follows.

**[Table 11]**

| Strength of Reflective Light (Raw Intensity) | | Determination | | | | | |
|---|---|---|---|---|---|---|---|
| | | Pass | PD1 | PD2 | PD3 | PD4 | Rem |
| Actual | Pass | 36 | 0 | 0 | 1 | 3 | 0 |
| | PD1 | 1 | 59 | 20 | 0 | 0 | 0 |
| | PD2 | 0 | 0 | 40 | 20 | 20 | 0 |
| | PD3 | 0 | 20 | 20 | 40 | 0 | 0 |
| | PD4 | 1 | 0 | 0 | 20 | 59 | 0 |
| | Rem | 2 | 3 | 0 | 0 | 1 | 0 |

Based on the Table 11, the result of the experiment is summed up as follows.

**[Table 12]**

| | | Determination | |
|---|---|---|---|
| | | Positive | Negative |
| Actual | Positive | 90.0% | 10.0% |
| | Negative | 1.1% | 98.9% |

For example, when a part of the user's body appropriately covers the sensor 170, cases where the processor 110 determines that the user's body appropriately covers the sensor 170 (Actual: positive - Determination: positive) may be 90.0%, and when a part of the user's body does not cover a part of the sensor 170, cases where the processor 110 determines that the user's body does not cover a part of the sensor 170 (Actual: negative - Determination: negative) may be 98.9%.

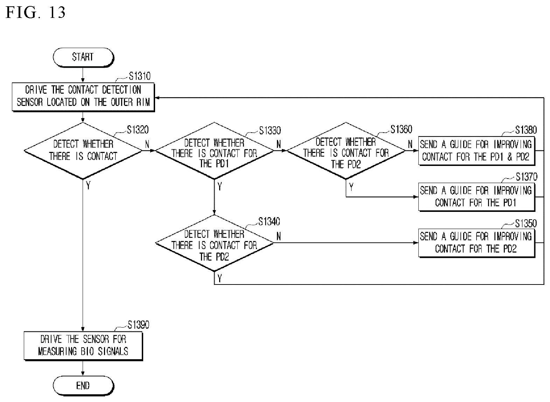

FIG. 13 is a flow chart for illustrating a control method of an electronic device according to an embodiment of the disclosure.

In the embodiments below, each operation may be performed sequentially, but they are not necessarily performed sequentially. For example, the order of each operation may be changed, or at least two operations may be performed in parallel.

According to an embodiment, operations S1310 to S1390 may be performed at a processor (e.g., the at least one processor 110 in FIG. 4) of an electronic device (e.g., the electronic device 100 in FIG. 4).

According to an embodiment, in a control method of the electronic device 100, if a user instruction controlling the electronic device 100 to measure the biometric information of the user is received in the operation S1310, the electronic device 100 may drive a contact detection sensor located on the outer rim (e.g., between the outside of the first area 1 and the inside of the second area 2). For example, the contact detection sensor may include at least one second LED 172 and a plurality of PDs 173 arranged between the outside of the first area 1 and the inside of the second area 2.

According to an embodiment, in the control method, it is detected whether there is appropriate contact between the contact detection sensor and the user's body in the operation S1320. For example, the electronic device 100 may detect whether the user's body covers the contact detection sensor (or, the sensor 170).

According to an embodiment, if appropriate contact between the contact detection sensor and the user's body was not detected (e.g., if it is identified that the user's body does not cover the contact detection sensor (or, the sensor 170)) in operations S1320-N and S1330, the electronic device 100 may detect whether there is contact for the first PD 173-1 for identifying whether the user's body covers the sensor 170.

For example, the electronic device 100 may control the 2-1 LED 172-1 adjacent to the first PD 173-1 to emit a light, and identify whether the user's body covers the first PD 173-1 based on first absorbance obtained as the first PD 173-1 absorbs some of the light emitted by the 2-1 LED 172-1.

According to an embodiment, if it is identified that the user's body covers the first PD 173-1 in the operations S1330-Y and S1340, the electronic device 100 may identify whether there is contact for the second PD 173-2.

For example, the electronic device 100 may control the 2-2 LED 172-2 adjacent to the second PD 173-2 to emit a light, and identify whether the user's body covers the second PD 173-2 based on second absorbance obtained as the second PD 173-2 absorbs some of the light emitted by the 2-2 LED 172-2.

According to an embodiment, if it is identified that the user's body covers the second PD 173-2 in the operation S1340-Y, the electronic device 100 may drive the contact detection sensor in the operation S1310, and if appropriate contact between the contact detection sensor and the user's body is detected in the operations S1320-Y and S1390 (e.g., if it is identified that the user's body covers the contact detection sensor (or, the sensor 170)), the electronic device 100 may drive a sensor for measuring bio signals. According to an embodiment, if it is identified that the user's body does not cover the second PD 173-2 in the operations S1340-N and S1350, the electronic device 100 may send a feedback (e.g., a guide UI) for improving contact between the user's body and the second PD 173-2.

According to an embodiment, if it is identified that the user's body does not cover the first PD 173-1 in the operations S1330-N and S1360, the electronic device 100 may detect whether there is contact for the second PD 173-2.

According to an embodiment, if it is identified that the user's body covers the second PD 173-2 in the operations S1360-Y and S1370, the electronic device 100 may send a feedback (e.g., a guide UI) for improving contact between the user's body and the first PD 173-1.

According to an embodiment, if it is identified that the user's body does not cover the first PD 173-1 and the second PD 173-2 in the operations S1360-N and S1380, the electronic device 100 may send a feedback (e.g., a guide UI) for improving contact between the user's body and each of the first PD 173-1 and the second PD 173-2.

According to an embodiment, in the control method, after sending a feedback in the operations S1350, S1370, and S1380, the electronic device 100 may drive the contact detection sensor in the operation S1310, and if appropriate contact between the contact detection sensor and the user's body is detected (e.g., if it is identified that the user's body covers the contact detection sensor (or, the sensor 170)) in the operations S1320-Y and S1390, the electronic device 100 may drive a sensor for measuring bio signals.

For example, a bio signal may include the concentration of antioxidative components, and the sensor for measuring bio signals may include a plurality of first LEDs 171 arranged inside the first area 1 and a plurality of PDs 173 arranged between the outside of the first area 1 and the inside of the second area 2.

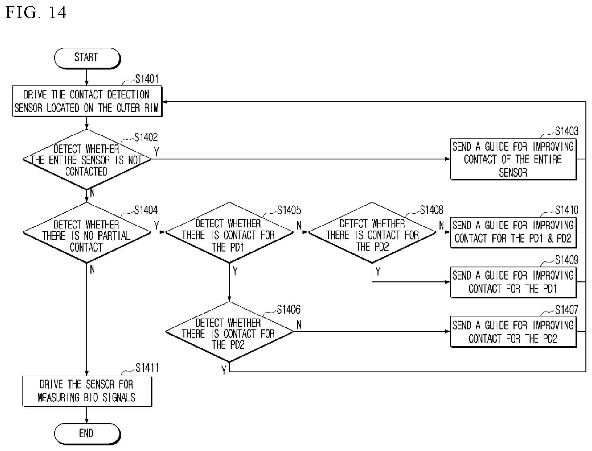

FIG. 14 is a flow chart for illustrating a control method of an electronic device according to an embodiment of the disclosure.

In the embodiments below, each operation may be performed sequentially, but they are not necessarily performed sequentially. For example, the order of each operation may be changed, or at least two operations may be performed in parallel.

According to an embodiment, operations S1401 to S1411 may be performed at a processor (e.g., the at least one processor 110 in FIG. 4) of an electronic device (e.g., the electronic device 100 in FIG. 4).

Referring to FIG. 14, in a control method of the electronic device 100, if a user instruction controlling the electronic device 100 to measure the biometric information of the user is received in the operation S1401, the electronic device 100 drives a contact detection sensor located on the outer rim (e.g., between the outside of the first area 1 and the inside of the second area 2).

According to an embodiment, if it is identified that the entire contact detection sensor and the user's body do not contact each other in the operations S1402-Y and S1403-Y, the electronic device 100 may send a feedback (e.g., a guide UI) guiding such that the user's body contacts the entire sensor 170.

According to an embodiment, if it is detected that there is a partial contact between the contact detection sensor and the user's body (e.g., if it is identified that the user's body covers a part of the contact detection sensor) in the operations S1402-N and S1404-Y, the electronic device 100 may detect whether there is contact for the first PD 173-1 for identifying whether the user's body covers an area corresponding to the first PD 173-1 in the sensor 170 in the operation S1405.

According to an embodiment, each of the operations S1405 to S1410 may correspond to each of the operations S1330 to S1380 illustrated in FIG. 13.

According to an embodiment, in the control method, after sending a feedback in the operations S1403, S1407, S1409, and S1410, the electronic device 100 may drive the contact detection sensor in the operation S1401, and if it is identified that the entire contact detection sensor contacts the user's body in the operations S1402-N, S1404-N, and S1411 (the operation S1402-N), and if it is identified that there is not an area that is not properly contacted by the user's body (or an area that is not covered by the user's body) in the sensor 170 (the operation S1404-N), the electronic device 100 may drive a sensor for measuring bio signals (the operation S1411).

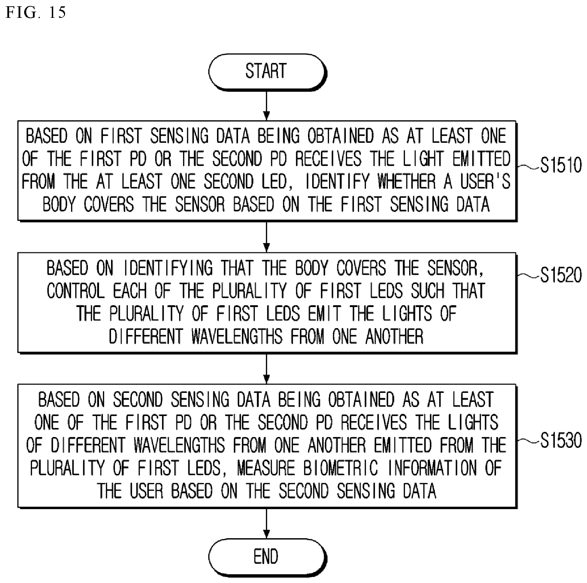

FIG. 15 is a flow chart for illustrating a control method of an electronic device according to an embodiment of the disclosure.

In the embodiments below, each operation may be performed sequentially, but they are not necessarily performed sequentially. For example, the order of each operation may be changed, or at least two operations may be performed in parallel.

According to an embodiment, operations S1510 to S1530 may be performed at a processor (e.g., the at least one processor 110 in FIG. 4) of an electronic device (e.g., the electronic device 100 in FIG. 4).

In a control method of an electronic device according to an embodiment, the electronic device may include a display arranged on the front surface of the electronic device, and a sensor arranged on the rear surface of the electronic device, and the sensor may include a plurality of first LEDs that are arranged inside a first area including a center of the sensor, and emit lights of different wavelengths from one another, at least one second LED that is arranged between the outside of the first area and the inside of a second area including the center, and a first photo diode (PD) and a second PD that are arranged between the outside of the first area and the inside of the second area, and receive lights emitted from the plurality of first LEDs and the at least one second LED, and are arranged to be symmetrical to each other based on the center.

In the control method according to an embodiment, if first sensing data is obtained as at least one of the first PD or the second PD receives the light emitted from the at least one second LED in the operation S1510, it is identified whether a user's body covers the sensor based on the first sensing data.

If it is identified that the body covers the sensor in the operation S1520, each of the plurality of first LEDs is controlled such that the plurality of first LEDs emit lights of different wavelengths from one another.

If second sensing data is obtained as at least one of the first PD or the second PD receives the lights of different wavelengths from one another emitted from the plurality of first LEDs in the operation S1530, biometric information of the user is measured based on the second sensing data.

The first sensing data according to an embodiment may include first absorbance obtained as the first PD received the light emitted from the at least one second LED and second absorbance obtained as the second PD received the light emitted from the at least one second LED, and the operation S1510 of identifying whether the body covers the sensor may include an operation of identifying whether the body covers the first PD and the second PD based on at least one of the first absorbance or the second absorbance.

The at least one second LED according to an embodiment may include a 2-1 LED arranged in a first direction and a 2-2 LED arranged in a second direction symmetrical to the first direction based on the center, and each of the 2-1 LED and the 2-2 LED may emit a light of a red or IR band, and the first PD may be arranged to be adjacent to the 2-1 LED, and the second PD may be arranged to be adjacent to the 2-2 LED.

The operation S1510 of identifying whether the body covers the sensor may include an operation of, based on the first absorbance obtained as the first PD received the light emitted from the 2-1 LED being greater than or equal to a threshold value, identifying that the body does not cover the first PD that obtained the first absorbance, and providing a feedback so as to induce covering of the first PD, and based on the second absorbance obtained as the second PD received the light emitted from the 2-2 LED being greater than or equal to the threshold value, identifying that the body does not cover the second PD that obtained the second absorbance, and providing the feedback so as to guide (or induce) covering of the second PD.

The operation of providing the feedback so as to guide (or induce) covering of the first PD may include an operation of, based on identifying that the body does not cover the first PD, providing a UI screen guiding a location of the body such that the body covers the first PD, and/or a sound notification and/or a vibration notification indicating the location of the first PD on the rear surface of the electronic device as the feedback, and the operation of providing the feedback so as to guide (or induce) covering of the second PD may include an operation of, based on identifying that the body does not cover the second PD, providing a UI screen guiding the location of the body such that the body covers the second PD, and/or a sound notification and/or a vibration notification indicating the location of the second PD on the rear surface as the feedback.

The method according to an embodiment may further include the operation of, based on receiving a user instruction controlling the electronic device to measure the biometric information, controlling the at least one second LED to emit a light after a predetermined time passes, and the operation S1530 of measuring the biometric information may include an operation of, while obtaining the first sensing data after the predetermined time passed, inactivating touch detection through the display.

The operation of controlling the at least one second LED to emit a light according to an embodiment may include an operation of, based on detecting a rotation of the electronic device such that the rear surface of the electronic device is upward through the motion sensor of the electronic device, controlling the at least one second LED to emit a light after the predetermined time passes, and the operation S1520 of controlling each of the plurality of first LEDs may include an operation of, based on identifying that the body covers the first PD and the second PD, controlling the plurality of first LEDs such that the plurality of first LEDs emit lights.

The operation S1530 of measuring the biometric information according to an embodiment may include an operation of, while obtaining the second sensing data, providing a UI screen, a sound notification, and/or a vibration notification indicating time left until measurement of the biometric information.

The biometric information according to an embodiment may include anti-oxidation concentration of the body.

The first area according to an embodiment may include a range of 2mm from the center, the second area may include a range of 20mm from the center, and the at least one second LED, the first PD, and the second PD may be arranged within a range of between 2mm and 20mm from the center, and the first PD may be arranged within a range of 6mm from a 2-1 LED included in the at least one second LED, and the second PD may be arranged within a range of 6mm from a 2-2 LED included in the at least one second LED.

According to an embodiment, an electronic device (e.g., the electronic device 100 in FIG. 2) includes a display arranged on the front surface of the electronic device (e.g., the display 140 in FIG. 2), a sensor arranged on the rear surface of the electronic device (e.g., the sensor 170 in FIG. 2), memory storing instructions, and including at least one storage medium (e.g., the memory 120 in FIG. 2), and at least one processor including processing circuitry (e.g., the processor 110 in FIG. 2), and the sensor includes a plurality of first LEDs that are arranged inside a first area including a center of the sensor, and emit lights of different wavelengths from one another, at least one second LED that is arranged between the outside of the first area and the inside of a second area including the center, and a first photo diode (PD) and a second PD that are arranged between the outside of the first area and the inside of the second area, and receive lights emitted from the plurality of first LEDs and the at least one second LED, and are arranged to be symmetrical to each other based on the center, and the at least one processor is configured to, based on first sensing data being obtained as at least one of the first PD or the second PD receives the light emitted from the at least one second LED, identify whether a user's body covers the sensor based on the first sensing data, and based on identifying that the body covers the sensor, control each of the plurality of first LEDs such that the plurality of first LEDs emit the lights of different wavelengths from one another, and based on second sensing data being obtained as at least one of the first PD or the second PD receives the lights of different wavelengths from one another emitted from the plurality of first LEDs, measure biometric information of the user based on the second sensing data.

For example, the first sensing data may include first absorbance obtained as the first PD received the light emitted from the at least one second LED and second absorbance obtained as the second PD received the light emitted from the at least one second LED.

For example, the at least one processor may be configured to identify whether the body covers the first PD and the second PD based on at least one of the first absorbance or the second absorbance.

For example, the at least one second LED may include a 2-1 LED arranged in a first direction and a 2-2 LED arranged in a second direction symmetrical to the first direction based on the center.

For example, each of the 2-1 LED and the 2-2 LED may emit a light of a red or IR band.

For example, the first PD may be arranged to be adjacent to the 2-1 LED, and the second PD may be arranged to be adjacent to the 2-2 LED.

For example, the at least one processor may be configured to, based on the first absorbance obtained as the first PD received the light emitted from the 2-1 LED being greater than or equal to a threshold value, identify that the body does not cover the first PD that obtained the first absorbance, and provide a feedback so as to guide (or induce) covering of the first PD.

For example, the at least one processor may be configured to, based on the second absorbance obtained as the second PD received the light emitted from the 2-2 LED being greater than or equal to the threshold value, identify that the body does not cover the second PD that obtained the second absorbance, and provide the feedback so as to guide (or induce) covering of the second PD.

For example, the at least one processor may be configured to, based on identifying that the body does not cover the first PD, provide a UI screen guiding a location of the body such that the body covers the first PD, and/or a sound notification and/or a vibration notification indicating the location of the first PD on the rear surface of the electronic device as the feedback.

For example, the at least one processor may be configured to, based on identifying that the body does not cover the second PD, provide a UI screen guiding the location of the body such that the body covers the second PD, and/or a sound notification and/or a vibration notification indicating the location of the second PD on the rear surface as the feedback.

For example, the at least one processor may, based on receiving a user instruction controlling the electronic device to measure the biometric information, control the at least one second LED to emit a light after a predetermined time passes.

For example, the at least one processor may, while obtaining the first sensing data after the predetermined time passed, inactivate touch detection through the display.

For example, the electronic device may further include a motion sensor.

For example, the at least one processor may, based on detecting a rotation of the electronic device such that the rear surface of the electronic device is upward through the motion sensor, control the at least one second LED to emit a light after the predetermined time passes.

For example, the at least one processor may, based on identifying that the body covers the first PD and the second PD, control the plurality of first LEDs such that the plurality of first LEDs emit lights.

For example, the at least one processor may, while obtaining the second sensing data, provide a UI screen, a sound notification, and/or a vibration notification indicating time left until measurement of the biometric information.

For example, the at least one processor may measure the biometric information of the user based on the second sensing data.

For example, the biometric information may include anti-oxidation concentration of the body.

For example, the first area may include a range of 2mm from the center.

For example, the second area may include a range of 20mm from the center.

For example, the at least one second LED, the first PD, and the second PD may be arranged within a range of between 2mm and 20mm from the center.

For example, the first PD may be arranged within a range of 6mm from a 2-1 LED included in the at least one second LED, and the second PD may be arranged within a range of 6mm from a 2-2 LED included in the at least one second LED.

In a control method of an electronic device (e.g., the electronic device 100 in FIG. 2) according to an embodiment, the electronic device includes a display arranged on the front surface of the electronic device (e.g., the display 140 in FIG. 2), and a sensor arranged on the rear surface of the electronic device (e.g., the sensor 170 in FIG. 2), and the sensor includes a plurality of first LEDs that are arranged inside a first area including a center of the sensor, and emit lights of different wavelengths from one another, at least one second LED that is arranged between the outside of the first area and the inside of a second area including the center, and a first photo diode (PD) and a second PD that are arranged between the outside of the first area and the inside of the second area, and receive lights emitted from the plurality of first LEDs and the at least one second LED, and are arranged to be symmetrical to each other based on the center.

The method includes the operations of, based on first sensing data being obtained as at least one of the first PD or the second PD receives the light emitted from the at least one second LED, identifying whether a user's body covers the sensor based on the first sensing data, and based on identifying that the body covers the sensor, controlling each of the plurality of first LEDs such that the plurality of first LEDs emit the lights of different wavelengths from one another, and based on second sensing data being obtained as at least one of the first PD or the second PD receives the lights of different wavelengths from one another emitted from the plurality of first LEDs, measuring biometric information of the user based on the second sensing data.

For example, the first sensing data may include first absorbance obtained as the first PD received the light emitted from the at least one second LED and second absorbance obtained as the second PD received the light emitted from the at least one second LED.

For example, the operation of identifying whether the body covers the sensor may include an operation of identifying whether the body covers the first PD and the second PD based on at least one of the first absorbance or the second absorbance.

For example, the at least one second LED may include a 2-1 LED arranged in a first direction and a 2-2 LED arranged in a second direction symmetrical to the first direction based on the center.

For example, each of the 2-1 LED and the 2-2 LED may emit a light of a red or an IR band.

For example, the first PD may be arranged to be adjacent to the 2-1 LED, and the second PD may be arranged to be adjacent to the 2-2 LED.

For example, the operation of identifying whether the body covers the sensor may include an operation of, based on the first absorbance obtained as the first PD received the light emitted from the 2-1 LED being greater than or equal to a threshold value, identifying that the body does not cover the first PD that obtained the first absorbance, and providing a feedback so as to guide (or induce) covering of the first PD, and an operation of, based on the second absorbance obtained as the second PD received the light emitted from the 2-2 LED being greater than or equal to the threshold value, identifying that the body does not cover the second PD that obtained the second absorbance, and providing the feedback so as to guide (or induce) covering of the second PD.

For example, the operation of providing the feedback so as to guide (or induce) covering of the first PD may include an operation of, based on identifying that the body does not cover the first PD, providing a UI screen guiding a location of the body such that the body covers the first PD, and/or a sound notification and/or a vibration notification indicating the location of the first PD on the rear surface of the electronic device as the feedback.

For example, the operation of providing the feedback so as to guide (or induce) covering of the second PD may include an operation of, based on identifying that the body does not cover the second PD, providing a UI screen guiding the location of the body such that the body covers the second PD, and/or a sound notification and/or a vibration notification indicating the location of the second PD on the rear surface as the feedback.

For example, the method may further include an operation of, based on receiving a user instruction controlling the electronic device to measure the biometric information, controlling the at least one second LED to emit a light after a predetermined time passes.

For example, the operation of measuring the biometric information may include an operation of, while obtaining the first sensing data after the predetermined time passed, inactivating touch detection through the display.

For example, the operation of controlling the at least one second LED to emit a light may include an operation of, based on detecting a rotation of the electronic device such that the rear surface of the electronic device is upward through the motion sensor of the electronic device, controlling the at least one second LED to emit a light after the predetermined time passes.

For example, the operation of controlling each of the plurality of first LEDs may include an operation of, based on identifying that the body covers the first PD and the second PD, controlling the plurality of first LEDs such that the plurality of first LEDs emit lights.

For example, the operation of measuring the biometric information may include an operation of, while obtaining the second sensing data, providing a UI screen, a sound notification, and/or a vibration notification indicating time left until measurement of the biometric information.

For example, the biometric information may include anti-oxidation concentration of the body.

For example, the first area may include a range of 2mm from the center, and the second area may include a range of 20mm from the center.

For example, the at least one second LED, the first PD, and the second PD may be arranged within a range of between 2mm and 20mm from the center.

For example, the first PD may be arranged within a range of 6mm from a 2-1 LED included in the at least one second LED, and the second PD may be arranged within a range of 6mm from a 2-2 LED included in the at least one second LED.

Meanwhile, the various embodiments described above may be implemented in a recording medium that may be read by a computer or a device similar to a computer, by using software, hardware, or a combination thereof. In some cases, the embodiments described in this specification may be implemented as a processor itself. According to implementation by software, the embodiments such as procedures and functions described in this specification may be implemented as separate software modules. Each of the software modules may perform one or more functions and operations described in this specification.

Meanwhile, computer instructions for performing processing operations of an electronic device according to the various embodiments of the disclosure described above may be stored in a non-transitory computer-readable medium. Computer instructions stored in such a non-transitory computer-readable medium make the processing operations at the electronic device according to the various embodiments described above performed by a specific machine, when the instructions are executed by the processor of the specific machine.

A non-transitory computer-readable medium refers to a medium that stores data semi-permanently, and is readable by machines, but not a medium that stores data for a short moment such as a register, a cache, and memory. As specific examples of a non-transitory computer-readable medium, there may be a CD, a DVD, a hard disc, a blue-ray disc, a USB, a memory card, ROM, and the like.

Also, while example embodiments of the disclosure have been shown and described, the disclosure is not limited to the aforementioned specific embodiments, and it is apparent that various modifications may be made by those having ordinary skill in the technical field to which the disclosure belongs, without departing from the gist of the disclosure as claimed by the appended claims. Further, it is intended that such modifications are not to be interpreted independently from the technical idea or prospect of the disclosure.

## Claims

1. An electronic device comprising:
a display arranged on a front surface of the electronic device;
a sensor arranged on a rear surface of the electronic device;
a memory storing instructions, and including at least one storage medium; and
at least one processor including at least one processing circuitry,
wherein the sensor comprises:
a plurality of first light emitting diodes (LEDs) that are arranged inside a first area including a center of the sensor, and emit lights of wavelengths different from one another;
at least one second LED that is arranged between an outside of the first area and an inside of a second area including the center; and
a first photo diode (PD) and a second PD that are arranged between the outside of the first area and the inside of the second area, configured to receive lights emitted from the plurality of first LEDs and the at least one second LED and are arranged to be symmetrical to each other based on the center, and
wherein the at least one processor is configured to:
based on first sensing data obtained by at least one of the first PD or the second PD that receives a light emitted from the at least one second LED, identify whether a body of a user covers the sensor;
based on identifying that the body covers the sensor, control each of the plurality of first LEDs to emit the lights of different wavelengths; and
based on second sensing data obtained by at least one of the first PD or the second PD that receives the lights of different wavelengths emitted from the plurality of first LEDs, measure biometric information of the user.

2. The electronic device of claim 1, wherein the first sensing data comprises:
a first absorbance obtained based on the first PD that receives the light emitted from the at least one second LED and a second absorbance obtained based on the second PD that receives the light emitted from the at least one second LED, and
wherein the at least one processor is configured to:
identify whether the body covers the first PD and the second PD based on at least one of the first absorbance or the second absorbance.

3. The electronic device of claim 2, wherein the at least one second LED includes a 2-1 LED arranged in a first direction and a 2-2 LED arranged in a second direction symmetrical to the first direction based on the center,
wherein each of the 2-1 LED and the 2-2 LED emits a light of a red color band or an infrared (IR) band,
wherein the first PD is arranged to be adjacent to the 2-1 LED, and
wherein the second PD is arranged to be adjacent to the 2-2 LED.

4. The electronic device of claim 3, wherein the at least one processor is configured to:
based on the first absorbance obtained based on the first PD that receives the light emitted from the 2-1 LED being greater than or equal to a threshold value, identify that the body does not cover the first PD, and provide a feedback thereon; and
based on the second absorbance obtained based on the second PD that receives the light emitted from the 2-2 LED being greater than or equal to the threshold value, identify that the body does not cover the second PD, and provide a feedback thereon.

5. The electronic device of claim 4, wherein the at least one processor is configured to:
based on identifying that the body does not cover the first PD, provide at least one of a user interface (UI) screen, a sound notification, or a vibration notification indicating at least one of a location of the body or a location of the first PD on the rear surface of the electronic device as the feedback, and/or
based on identifying that the body does not cover the second PD, provide at least one of a UI screen, a sound notification, or a vibration notification indicating at least one of a location of the body or a location of the second PD on the rear surface as the feedback.

6. The electronic device of claim 1, wherein the at least one processor is configured to:
based on receiving a user instruction to measure the biometric information, control the at least one second LED to emit a light after an elapse of a predetermined time; and
while obtaining the first sensing data after the elapse of the predetermined time, inactivate touch detection through the display.

7. The electronic device of claim 6, further comprising:
a motion sensor,
wherein the at least one processor is configured to:
based on detecting, through the motion sensor, a rotation of the electronic device such that the rear surface of the electronic device is upward, control the at least one second LED to emit a light after the elapse of the predetermined time; and
based on identifying that the body covers the first PD and the second PD, control the plurality of first LEDs to emit the lights.

8. The electronic device of claim 6, wherein the at least one processor is configured to:
while obtaining the second sensing data, provide at least one of a UI screen, a sound notification, or a vibration notification indicating a time left until measurement of the biometric information.

9. The electronic device of claim 1, wherein the at least one processor is configured to:
measure the biometric information of the user based on the second sensing data; and
the biometric information includes anti-oxidation concentration of the body.

10. The electronic device of claim 1, wherein the first area includes a range of 2mm from the center,
wherein the second area includes a range of 20mm from the center,
wherein the at least one second LED, the first PD, and the second PD are arranged within a range of between 2mm and 20mm from the center,
wherein the first PD is arranged within a range of 6mm from a first LED included in the at least one second LED, and
wherein the second PD is arranged within a range of 6mm from a second LED included in the at least one second LED.

11. A control method of an electronic device, the electronic device comprising: a display arranged on a front surface of the electronic device; a sensor arranged on a rear surface of the electronic device, and the sensor comprising: a plurality of first light emitting diodes (LEDs) that are arranged inside a first area including a center of the sensor, and emit lights of wavelengths different from one another, at least one second LED that is arranged between an outside of the first area and an inside of a second area including the center, and a first photo diode (PD) and a second PD that are arranged between the outside of the first area and the inside of the second area, configured to receive lights emitted from the plurality of first LEDs and the at least one second LED, and are arranged to be symmetrical to each other based on the center,
the method comprising:
based on first sensing data obtained by at least one of the first PD or the second PD that receives a light emitted from the at least one second LED, identifying whether a body of a user covers the sensor;
based on identifying that the body covers the sensor, controlling each of the plurality of first LEDs to emit the lights of different wavelengths; and
based on second sensing data obtained by at least one of the first PD or the second PD that receives the lights of different wavelengths emitted from the plurality of first LEDs, measuring biometric information of the user based on the second sensing data.

12. The control method of claim 11, wherein the first sensing data comprises:
first absorbance obtained as the first PD received the light emitted from the at least one second LED and second absorbance obtained as the second PD received the light emitted from the at least one second LED, and
wherein the identifying whether the body covers the sensor comprises:
identifying whether the body covers the first PD and the second PD based on at least one of the first absorbance or the second absorbance.

13. The control method of claim 12, wherein the at least one second LED includes a 2-1 LED arranged in a first direction and a 2-2 LED arranged in a second direction symmetrical to the first direction based on the center,
wherein each of the 2-1 LED and the 2-2 LED emits a light of a red or IR band,
wherein the first PD is arranged to be adjacent to the 2-1 LED, and
wherein the second PD is arranged to be adjacent to the 2-2 LED.

14. The control method of claim 13, wherein the identifying whether the body covers the sensor comprises:
based on the first absorbance obtained based on the first PD that receives the light emitted from the first LED being greater than or equal to a threshold value, identifying that the body does not cover the first PD, and providing a feedback thereon; and
based on the second absorbance obtained based on the second PD that receives the light emitted from the second LED being greater than or equal to the threshold value, identifying that the body does not cover the second PD, and providing a feedback thereon.

15. The control method of claim 14, wherein the providing the feedback with respect to the first PD comprises:
based on identifying that the body does not cover the first PD, providing at least one of a UI screen, a sound notification, or a vibration notification indicating at least one of a location of the body or a location of the first PD on the rear surface of the electronic device as the feedback, and
wherein the providing the feedback with respect to the second PD comprises:
based on identifying that the body does not cover the second PD, providing at least one of a UI screen, a sound notification, or a vibration notification indicating at least one of a location of the body or a location of the second PD on the rear surface as the feedback.
